# EUROPEAN PATENT SPECIFICATION

(11) **EP 4 522 241 B1**
(45) Date of publication and mention of the grant of the patent: **09.09.2026**
(21) Application number: 23836094.5
(22) Date of filing: 06.07.2023
(51) Int. Cl.: A61M 1/06, A61H 15/00

(54) **WEARABLE SYSTEM FOR BREAST PUMPING WITH MASSAGE**
TRAGBARES SYSTEM ZUM BRUSTPUMPEN MIT MASSAGE
SYSTÈME POUVANT ÊTRE PORTÉ POUR LE POMPAGE DU SEIN AVEC MASSAGE

(30) Priority: 07.07.2022 US 202263359056 P
(43) Date of publication of application: 19.03.2025
(73) Proprietor: Imalac, Inc., Miami, FL 33156 (US)
(72) Inventor: SABLOTSKY, Noreen Gordon, Coral Gables, Florida 33146 (US); KISH, Rachael Sablotsky, Miami, Florida 33158 (US); SABLOTSKY, Chelsea Alysa, Miami, Florida 33133 (US); WILSON, Zachary, Orlando, Florida 32826 (US); CRUZ, Roberto, Apopka, Florida 32703 (US); PALMER, Andrew, Winter Springs, Florida 32708 (US)
(74) Representative: Murgitroyd & Company
(86) International application number: PCT/US2023/027021
(87) International publication number: WO 2024/010865

(56) References cited:
- WO-A1-2021/207258
- US-A1- 2008 262 420
- US-A1- 2017 112 983
- US-A1- 2017 112 983
- US-A1- 2017 368 244
- US-A1- 2018 132 541
- US-A1- 2019 381 225
- US-A1- 2020 129 691
- US-B1- 6 440 100
- US-B1- 8 484 763

## Description

This disclosure claims priority to US Provisional Appl. No. 63/359,056, filed on July 7, 2022.

### BACKGROUND

### 1. Field

This disclosure relates to breast milk expression apparatus and systems.

### 2. State of the Art

Women's breasts are made of specialized milk-producing tissues including glandular as well as fatty tissues. The milk-producing part of the breast is organized into 15 to 20 sections, called lobes. Within each lobe are smaller structures, called lobules, where milk is produced. The milk travels through a network of tiny tubes called ducts. The ducts connect and come together into larger ducts, which eventually exit the skin in the nipple. There is a considerable body of evidence in the literature on the proven benefits of breast massage during lactation. These benefits include, but are not limited to: i) increase in volume of milk expressed due to more complete emptying; ii) increase in both nutritional and caloric value of milk due to increase in fat content of expressed milk; iii) decrease in time required for pumping; and iv) decrease in clogged milk ducts and engorgement.

The following documents may provide technical background to the present disclosure: US2019/0381225 A1, which discloses the preamble of claim 1, directed to a device that provides for mechanical stimulation of breasts of a user to promote milk expression; US2017/112983 A1 which discloses the use of a cable (see FIG. 12) coupled to a bra worn by a user, where the cable is disposed about the breast of the user and moves to provide for mechanical stimulation of the breast to promote milk expression; US6440100 B1 directed to a concealed apparatus for hands free breast milk pumping and storage; and US2008/262420 A1 directed to a breast pump device with self-contained breast milk reservoir.

### SUMMARY

As will be appreciated from the following description, in accordance with at least one aspect, a breast milk expression apparatus and system can replace manual (by hand) milk extraction.

The claimed invention provides a breast milk expression apparatus as defined in appended claim 1. Optional features are defined in the appended dependent claims.

In embodiments, the pocket is formed from fabric that is knitted or sewn or otherwise joined to or integrated into the bra.

In embodiments, the housing can include a removable door for installing or replacing the at least one battery.

In embodiments, the supporting electronics can include electrical circuitry configured to charge the at least one battery using DC power supplied by an external DC power source.

In embodiments, the housing can further enclose a wireless antenna and electronics configured to provide wireless communication between the unit and another device for controlling operation of the unit.

In embodiments, the housing can further enclose a vacuum pump powered by electrical power supplied by the at least one battery. The vacuum pump can provide suction to an external suction head and bottle assembly for one or both of the left and right breast of the user during the compression-expansion cycles.

In embodiments, the housing can further enclose a solenoid valve configured to selectively vent to atmosphere to release suction.

In embodiments, the first and second massage actuators each include a rotary pull mechanism.

In embodiments, the first and second massage actuators can include rotary pull mechanisms driven by the electric motor. The rotary pull mechanisms can employ multi-part winding spools with gears that interface to a worm gear that is rotatably driven by the electric motor. At least part of the first plurality of cable loops and at least part of the second plurality of cable loops can terminate with elements (e.g., beads) that are captured by corresponding scoop features of the multi-part winding spools. Certain scoop features of the multi-part winding spools can be offset from one another at predefined angles of rotation for sequential winding operations by the multi-part winding spools.

In embodiments, first tubing can be provided for the first plurality of cable loops. The first tubing can extend through or be supported by fabric disposed along a left-wing panel of the bra. Second tubing can be provided for the second plurality of cable loops. The second tubing can extend through or be supported by fabric disposed along a right-wing panel of the bra. The housing can further include a first cable connector disposed adjacent to the first massage actuator and a second cable connector disposed adjacent the second massage actuator. The first tubing can terminate at a connector that is configured to selectively mate and lock to the first cable connector. The second tubing can terminate at a connector that is configured to selectively mate and lock to the second cable connector.

In embodiments, the first pad can include sets of interlocking bead-like structures that define passageways for sections of the first plurality of cable loops, and the second pad can include sets of interlocking bead-like structures that define passageways for sections of the second plurality of cable loops. The interlocking bead-like structures of the first set can include interior spring elements that aid in expansion of the first plurality of cable loops. The interlocking bead-like structures of the second set can include interior spring elements that aid in expansion of the second plurality of cable loops.

Other aspects and features are described and claimed.

### BRIEF DESCRIPTION OF THE DRAWINGS

The foregoing will be apparent from the following more particular description of example embodiments of the invention, as illustrated in the accompanying drawings in which like reference characters refer to the same parts throughout different views. The drawings are not necessarily to scale, emphasis being placed on illustrating embodiments of the invention.
FIG. 1 is a schematic front view of a bra that supports left and right massage units.
FIG. 2 is a schematic rear view of the bra of FIG. 1.
FIG. 3 is a schematic left side view of the bra of FIG. 1.
FIG. 4 is a schematic front view illustrating the bra of FIG. 1 worn by a user.
FIG. 5 is a schematic rear view illustrating the bra of FIG. 1 worn by a user.
FIG. 6 is a schematic left side view of the bra of FIG. 1 worn by a user.
FIG. 7 is a perspective view of an exemplary left breast massage unit that can be supported by the bra of FIG. 1.
FIG. 8 is a schematic diagram of the massage unit of FIG. 7.
FIGS. 9A and 9B are schematic views illustrating internal components of the removable part of the massage unit of FIG. 7.
FIGS. 9C, 9D, 9E, and 9F are schematic views illustrating internal components of the on-pad part of massage unit of FIG. 7.
FIG. 10 is a perspective view of another exemplary massage unit that can be supported by the bra of FIG. 1.
FIG. 11A is a schematic view of yet another exemplary massage unit that can be supported by the bra of FIG. 1.
FIGS. 11B, 11C, and 11D are schematic views illustrating internal components of the removable part of the massage unit of FIG. 11A.
FIG. 12 is a perspective view of yet another exemplary massage unit that can be supported by the bra of FIG. 1.
FIG. 13 is a schematic view of still another exemplary massage unit that can be supported by the bra of FIG. 1.
FIG. 14 is a schematic front view of an exemplary bra that supports a single massage unit.
FIG. 15 is a schematic left side view of the bra of FIG. 14.
FIG. 16 is a schematic rear view of the bra of FIG. 14.
FIG. 17 is a schematic front view illustrating the bra of FIG. 14 worn by a user.
FIG. 18 is a schematic rear view illustrating the bra of FIG. 14 worn by a user.
FIG. 19 is a schematic left side view of the bra of FIG. 14 worn by a user.
FIG. 20 is a perspective front-side view of an exemplary massage unit that can be supported by the bra of FIG. 14.
FIG. 21 is a perspective rear-side view of the massage unit of FIG. 20.
FIG. 22A is a schematic view illustrating internal components of massage unit of FIG. 20.
FIG. 22B is a schematic diagram of the massage unit of FIG. 20.
FIGS. 23 to 29 are schematic views illustrating components of the massage unit of FIG. 20.
FIG. 30 is a schematic front view of another exemplary bra that incorporates interlocking bead-like elements for applying cyclical massage to the left and right breasts of a user.
FIGS. 31 to 34 are schematic views illustrating components of the interlocking bead-like elements used in the bra of FIG. 30.
FIG. 35 is a schematic diagram of an exemplary computer system that can embody parts of the breast massage units and corresponding control system or computing device as described herein.

### DETAILED DESCRIPTION OF THE PREFERRED EMBODIMENTS

It is to be understood that the present disclosure provides many different embodiments, or examples, for implementing different features of various embodiments. Specific examples of components and arrangements are described below to simplify the present disclosure. These are, of course, merely examples and are not intended to be limiting.

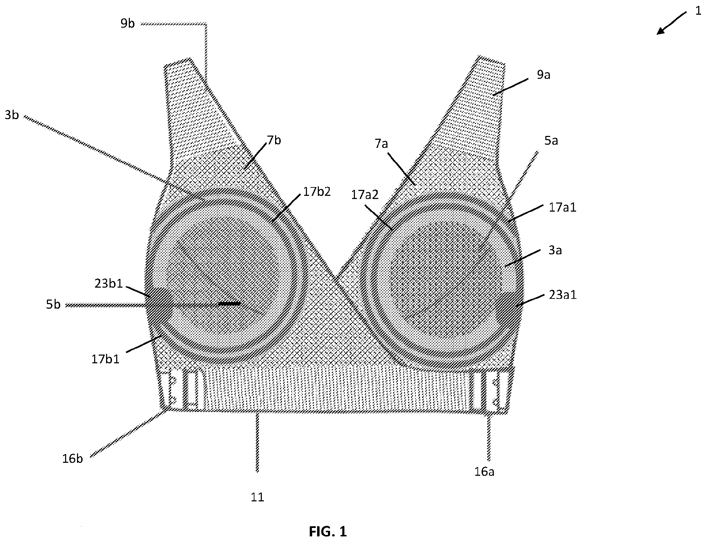

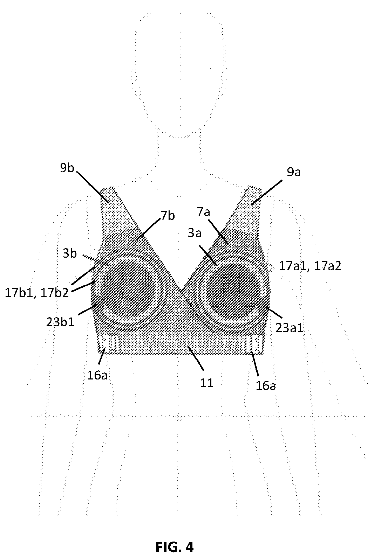

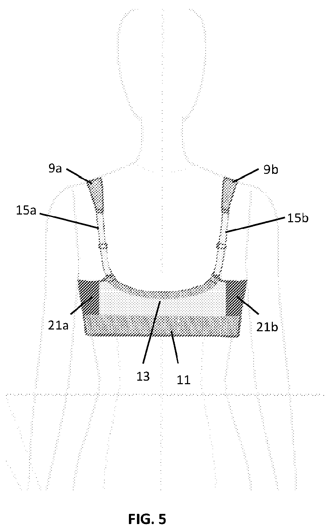

FIGS. 1 to 8 show one embodiment of a system that includes left and right massage units that are removably supported by a bra 1.

The bra 1 includes a pad 3a that is contoured and configured to surround and engage the left breast of a user during use. For purposes herein, the term "pad" is to be understood broadly to include one or pads or cups or shells that is contoured and configured to surround and engage a user's breast. The pad 3a may be made from fabric, polymer, or any flexible material. The pad 3a defines an opening or slit 5a corresponding to the nipple area of the left breast. The opening or slit 5a can be configured to receive therethrough a portion of a breast shield (not shown). The opening or slit 5a can be defined by overlapping fabric (e.g., with one-half inch of overlapping fabric). The pad 3a is supported by a tapered left panel 7a of the bra 1, which extends from a left upper strap 9a down to a bottom band 11, which is disposed below the pad 3a and surrounds the torso of the user. The left upper strap 9a is configured to extend over the left shoulder of the user and connects to a back panel 13 by an adjustable elastic strap 15a. The bottom band 11 can be formed from an elastic fabric material. The bottom band 11 can include a first set of fasteners 16a (e.g., hook and eye fasteners or hook and loop or Velcro fasteners) disposed on or near the left side of the bra 1 to enable opening and closing the bottom band 11 for putting on and removing the bra 1 during use.

The bra 1 also includes a pad 3b that is contoured and configured to surround and engage the right breast of a user during use. The pad 3b may be made from fabric, polymer, or any flexible material. The pad 3b defines an opening or slit 5b corresponding to the nipple area of the right breast. The opening or slit 5b can be configured to receive therethrough a portion of a breast shield (not shown). The opening or slit 5b can be defined by overlapping fabric (e.g., with one-half inch of overlapping fabric). The pad 3b is supported by a tapered right panel 7b of the bra 1, which extends from a right upper strap 9b down to the bottom band 11. The bottom band 11 can include a second set of fasteners 16b (e.g., hook and eye fasteners) disposed on or near the right side of the bra 1 to enable opening and closing the bottom band 11 for putting on and removing the bra 1 during use. The right upper strap 9b is configured to extend over the right shoulder of the user and connects to the back panel 13 by an adjustable elastic strap 15a.

The left strap 15a can employ connectors 20a (such as bar tacks and/or triangular sliders) to connect the left strap 15a to the left upper strap 9a and to the back panel 13. The left strap 15a can also include one or more adjusters 19a (such as a slider) to enable the length of the left strap 15a to be adjusted by the user. The right strap 15b can employ connectors 20b (such as bar tacks and/or triangular sliders) to connect the right strap 15b to the right upper strap 9b and to the back panel 13. The right strap 15b can also include one or more adjusters 19b (such as a slider) to enable the length of the right strap 15b to be adjusted by the user. The back panel 13 extends upward from the bottom band 11 to connect to the left and right straps 15a, 15b.

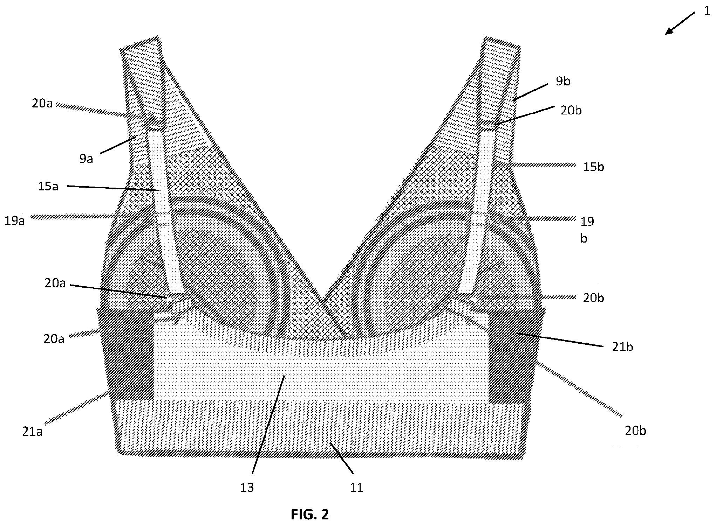

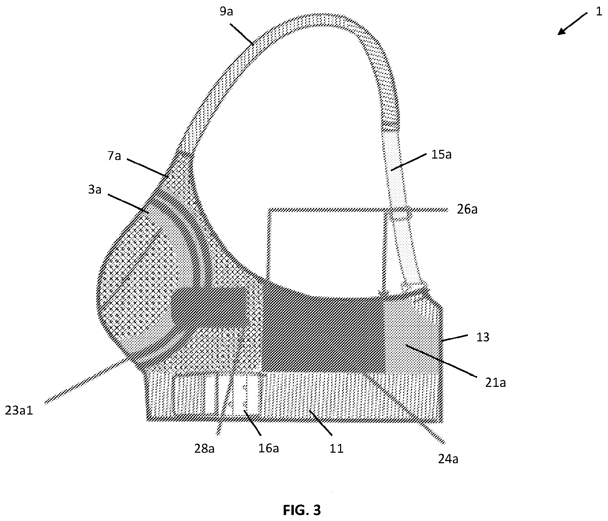

As best shown in FIGS. 2 and 3, bra 1 also includes a left-wing panel 21a that extends from the left side of panel 7a rearwards to the back panel 11. The left-wing panel 21a is configured to extend about the left side of the torso of the user and under the left arm of the user during use. The bra 1 also includes a right-wing panel 21b that extends from the right side of panel 7b to the back panel 13 similar to the left-wing panel 21a. The right-wing panel 21b is configured to extend about the right side of the torso of the user and under the right arm of the user during use.

The panels 7a, 7b, 13, 21a, 21b and the left and right upper straps 15a, 15b and the bottom band 11 can be formed from fabric, polymer, or any flexible material. In embodiments, the panels 7a, 7b, 13, 21a, 21b as well as the pads 3a, 3b and bottom band can be formed by a three-dimensional knitting machine programmed via a digital design of the bra and supplied with desired yarn and/or other suitable material. In embodiments, the whole bra can be made of the same basic material, such as jacquard knit, with possible variations in the amount of latex or other material used in certain areas or parts of the bra for desired properties (such as elasticity or comfort).

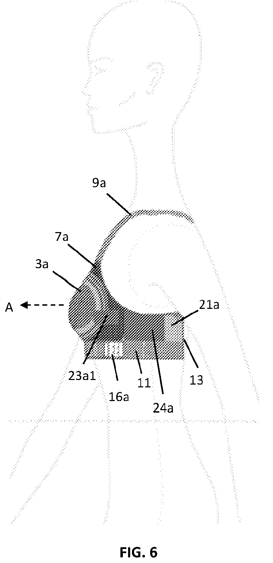

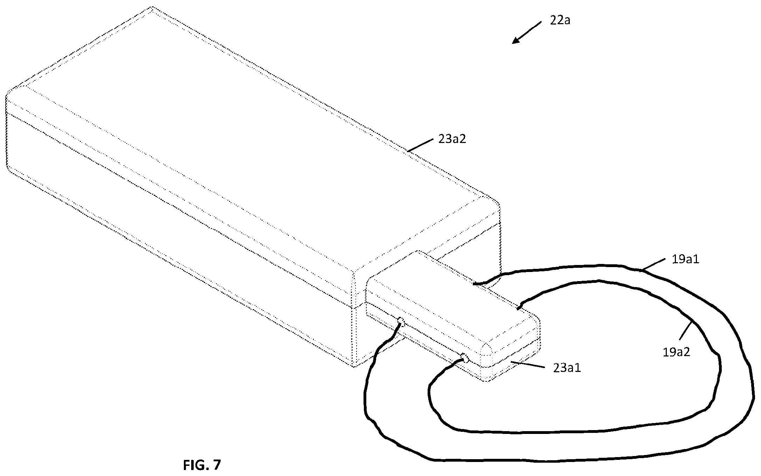

The pad 3a supports a plurality of cables configured to slide, translate, or otherwise displace relative to the pad 3a along circumferential paths guided by an arrangement of channels that are formed integral to the pad 3a. In FIGS. 1 to 3, two channels are shown as solid lines and labeled 17a1 and 17a2 with cables 19a1, 19a2 (FIG. 7) extending therethrough. In embodiments, the channels 17a1, 17a2 can be formed from fabric material knitted, sewn or otherwise joined to the pad 3a, such as a tubular jacquard knit. The cables that extend through channels 17a1, 17a2 are referred to as "cable loops" herein. For purposes herein, the term "cable" is to be understood broadly to include wire, string, monofilaments, multifilaments, twisted filaments, etc., which may be comprised of any of several materials such as metal, polymers which are substantially inelastic in tension, or other suitable materials, or combinations thereof. The circumferential paths formed by the channels 17a1, 17a2 (and the corresponding cable loops extending therethrough) are offset from one another in a longitudinal direction A as best shown in FIG. 6. During use, the longitudinal direction A is intended to extend generally parallel to the sagittal plane of the user from the torso/base of the left breast to the nipple of the left breast. In this manner, the circumferential paths formed by the channels 17a1, 17a2 (and the corresponding cable loops extending therethrough) are spaced from one another at different positions relative to the base of the left breast or torso of the user.

The pad 3b supports a plurality of cables configured to slide, translate, or otherwise displace relative to the pad 3b along circumferential paths guided by an arrangement of channels that are formed integral to the pad 3b. In FIGS 1 to 3, two channels are shown as solid lines and labeled 17b1 and 17b2 with cables (similar to cables 19a1, 19a2 of FIG. 7) extending therethrough. In embodiments, the channels 17b1, 17b2 can be formed from fabric material knitted, sewn or otherwise joined to the pad 3b, such as a tubular jacquard knit. The cables that extend through channels 17b1, 17b2 are referred to as "cable loops" herein. The circumferential paths formed by the channels 17b1, 17b2 (and the corresponding cable loops extending therethrough) are offset from one another in a longitudinal direction similar to direction A shown in FIG. 6. During use, the longitudinal direction is intended to extend generally parallel to the sagittal plane of the user from the torso/base of the right breast to the nipple of the right breast. In this manner, the circumferential paths formed by the channels 17b1, 17b2 (and the corresponding cable loops extending therethrough) are spaced from one another at different positions relative to the base of the right breast or torso of the user.

### LEFT MASSAGE UNIT

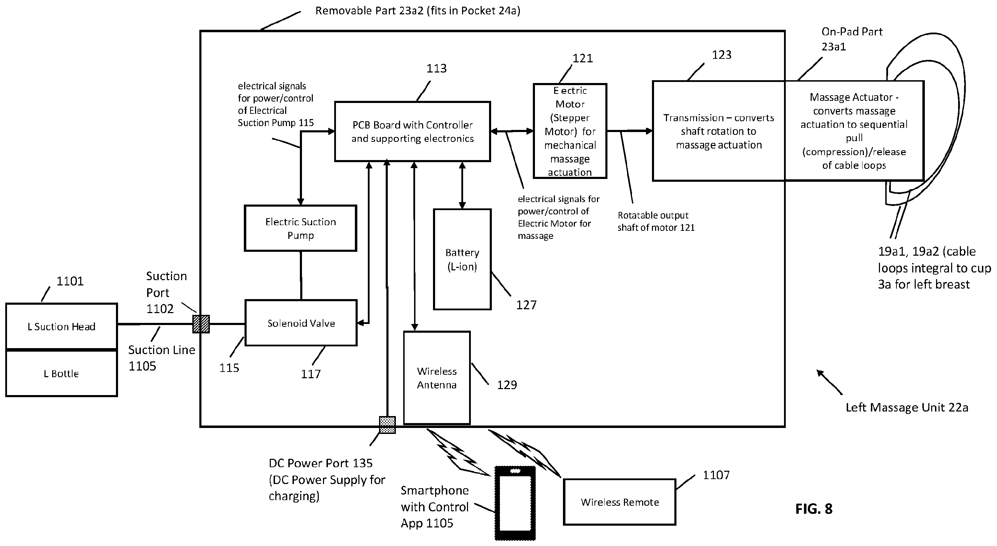

The cable loops 19a1, 19a2 that extend through the channels 17a1, 17a2 of pad 3a are mechanically coupled to a left massage unit 22a as best shown in FIG. 7. The left massage unit 22a can be configured to actuate displacement of the cable loops 19a1, 19a2 relative to the pad 3a to compress the left breast, and also reverse the displacement of the cable loops 19a1, 19a2 relative to the pad 3a to permit the left breast to expand and decompress. The left massage unit 22a employs an assembly that includes an on-pad part 23a1 and a removable part 23a2. As shown in FIG. 8, the removable part 23a2 houses a PCB 113 with a controller and supporting electronics, an electric motor (e.g., stepper motor) 121, at least one battery 127 (e.g., lithium-ion battery), and a mechanical transmission 123 (e.g., rotating driveshaft and possibly additional parts) driven by the electric motor 121. The removable part 23a2 can also house an electric suction pump 115 and a solenoid valve 117. The electric suction pump 115 is used to supply suction to an external suction head 1101 that is configured to surround the nipple of the left breast and aid in expressing milk from the left breast. The on-pad part 23a1 houses a massage actuator that interfaces to the cable loops 19a1, 19a2. The massage actuator of the on-pad part 23a1 is operably coupled to the mechanical transmission of the removable part 23a2. The user can selectively connect and disconnect the removable part 23a2 to and from the on-pad part 23a1 as desired. The operation of the massage actuator of the on-pad part 23a1 is mechanically driven by the transmission 123 of the removable part 23a2.

In embodiments, the electric motor 121 and transmission 123 of the removable part 23a2 can cooperate mechanically with the massage actuator of the on-pad part 23a1 to apply one or more compression-expansion cycles to the cable loops 19a1, 19a2. Each compression-expansion cycle selectively displaces the cable loops 19a1, 19a2 to decrease the effective lengths of the cable loops 19a1, 19a2 that surround the left breast to compress the left breast, and then displaces the cable loops 19a1, 19a2 to increase the effective lengths of the cable loops 19a1, 19a2 that surround the left breast to permit the breast to expand and decompress. In embodiments, each compression-expansion cycle includes compression operations that sequentially displace the cable loops 19a1, 19a2 to decrease the effective lengths of the cable loops 19a1, 19a2 that surround the left breast from the inner cable loop 19a1 (closer to torso) to the outer cable loop 19a2 (furthest from torso) without removal of tension from the cable loops in order to apply sequential compression to the left breast. Each compression-expansion cycle further includes expansion operations that displace the cable loops 19a1, 19a2 to increase the effective lengths of the cable loops 19a1, 19a2 that surround the left breast to permit expansion of the left breast. The sequential compression operations followed by the expansion operations as part of the compression-expansion cycle mimics massaging of the left breast and can effectively move milk toward the nipple of the breast for expression therefrom.

In embodiments, the controller and supporting electronics of PCB 113 of the removable part 23a2 can be configured to supply electrical signals to the electric motor 121 to provide power and control of the electric motor 121 in driving the mechanical massage actuation of the left breast as provided by cooperation of the motor 121, the transmission 123 and the massage actuator of the left massage unit 22a. The controller and supporting electronics of the PCB 113 of the removable part 23a2 can also be configured to supply electrical signals to the electric suction pump 115 of the removable part 23a2 to control the operation of the electric suction pump 115 in supplying suction to the external suction head and bottle assembly 1101 via a suction port 1102 in the removable part 23a2. A suction line 1105 can be fluidly coupled to this suction port 1102 to connect to the external suction head and bottle assembly 1101. In this configuration, the suction line 1105 supplies negative pressure (suction) generated by the electric suction pump 115 of the removable part 23a2 to the external suction head and bottle assembly 1101. The solenoid valve 117 can be configured to vent the suction line 1105 to the ambient environment under control of the controller of the PCB 113 as needed.

The removable part 23a2 of the left massage unit 22a can also support a wireless antenna 129 that interfaces to the controller and supporting electronics of the PCB 113 of the removable part 23a2. The controller and supporting electronics of the PCB 113 can cooperate with the wireless antenna 129 to provide a wireless communication link to a computing device. The wireless communication link can support a standardized wireless communication protocol such as any one of a number of Bluetooth^{®} protocols (e.g., Bluetooth^{®} v1.0 to v1.08, Bluetooth^{®} v1.1, Bluetooth^{®} v1.2, and Bluetooth^{®} v2.0), or any one of a number of Wi-Fi or IEEE 802.11 protocols, or other wireless data communication protocols. The computing device can be a smart phone, smart watch, tablet computer, or other computing device. For example, FIG. 8 depicts a smartphone labeled 1105. Alternatively or additionally, the computing device can be a wireless remote device, which is labeled 1107 in FIG. 8. The computing device may execute software (e.g., an application, a.k.a., an "app") that graphically displays an operational interface to a user. The operational interface may be used to configure and/or control the operating parameters of the left massage unit 22a, including operating parameters of the electric motor 121 that drives the massage actuation of the left massage unit 22a and operating parameters of the electric suction pump 115 of the left massage unit 22a. The operating parameters may be saved locally on the computing device or remotely (for example, in cloud data storage) during use for retrieval and/or analysis.

Furthermore, the removable part 23a2 of the left massage unit 22a can include a port 135 for receiving DC electrical power that charges the one or more batteries 127 of the removable part 23a2. When charged, the one or more batteries 127 of the removable part 23a2 can be used for supply of electrical power to the electrical components of the removable part 23a2, including the controller and supporting electronics of the PCB 113, the electric motor 121, and the electric suction pump 115. Optionally, the DC electrical power can also provide electrical power to the electrical components of the removable part 23a2. The electrical power can be regulated for supply to the electrical components of the removable part 23a2 as needed.

In embodiments, the left-wing panel 21a of the bra 1 can include a pocket or sleeve 24a formed from fabric as shown in FIGS. 3 and 6. In embodiments, the fabric of the pocket or sleeve 24a can be a jacquard knit that is knitted or sewn or otherwise joined to the left-wing panel 21a. The dimensions and material of the pocket or sleeve 24a can be configured to enclose and secure at least part of the left massage unit 22a unit during use. For example, the dimensions and material of the pocket or sleeve 24a can be configured to retain and hold the removable part 23a2 of the left massage unit 22a. In this configuration, the bra 1 can support the removable part 23a2 of the left massage unit 22a in a position under the left arm of the user that is hidden (or at least partially hidden) from view during normal interaction with others, which enables the left massage unit 22a to be worn and used in a discrete manner. In embodiments, an opening 26a leading into the pocket or sleeve 24a extends laterally across the top side of the pocket or sleeve 24a, while a smaller vertical opening 28a leading into the pocket or sleeve 24a extends along the front side of the pocket or sleeve 24a. The opening 26a can be sized to accommodate the lengthwise dimension of the removable part 23a2 of the left massage unit 22a to enable the removable part 23a2 to be placed into the pocket or sleeve 24a or removed from the pocket or sleeve 24a. The opening 28a can be sized to accommodate the height dimension of the on-pad part 23a1 of the left massage unit 22a such that the on-pad part 23a1 extends through the opening 28a to a position at or near the pad 3a as best shown in FIGS. 3 and 6.

### RIGHT MASSAGE UNIT

The two cable loops that extend through channels 17b1, 17b2 of pad 3b are mechanically coupled to a right massage unit, which is similar to the left massage unit 22a of FIG. 7. The right massage unit can be configured to actuate displacement of the two cable loops relative to pad 3b to compress the right breast, and also reverse the displacement of the cable loops relative to pad 3b to permit the right breast to expand and decompress. The right massage unit can employ an assembly that includes an on-pad part and a removable part similar to the left massage unit 22a of FIGS. 7 and 8. In this configuration, the removable part of the right massage unit houses a PCB with a controller and supporting electronics, an electric motor (e.g., stepper motor), at least one battery (e.g., lithium-ion battery), and a mechanical transmission (e.g., rotating driveshaft and possibly additional parts) driven by the electric motor. The removable part of the right massage unit can also house an electric suction pump and a solenoid valve for venting the suction pump. The suction pump is used to supply suction to an external suction head that is configured to surround the nipple of the right breast and aid in expressing milk from the right breast. The on-pad part of the right massage unit houses a massage actuator that interfaces to the cable loops. The massage actuator of the on-pad part is operably coupled to the mechanical transmission of the removable part. The user can selectively connect and disconnect the removable part to and from the on-pad part as desired. The operation of the massage actuator of the on-pad part is mechanically driven by the mechanical transmission of the removable part of the right massage unit.

In embodiments, the electric motor and mechanical transmission of the removable part can cooperate mechanically with the massage actuator of the on-pad part of the right massage unit to apply one or more compression-expansion cycles to the cable loops. Each compression-expansion cycle selectively displaces the cable loops to decrease the effective lengths of the cable loops that surround the right breast to compress the right breast, and then displaces the cable loops to increase the effective lengths of the cable that surround the right breast to permit the breast to expand and decompress. In embodiments, each compression-expansion cycle includes compression operations that sequentially displace the cable loops to decrease the effective lengths of the cable loops that surround the right breast from the inner cable loop (closer to the torso) to the outer cable (furthest from the torso) without removal of tension from the cable loops in order to apply sequential compression to the right breast. Each compression-expansion cycle further includes expansion operations that displace the cable loops to increase the effective lengths of the cable loops that surround the right breast to permit expansion of the right breast. The sequential compression operations followed by the expansion operations as part of the compression-expansion cycle mimics massaging of the right breast and can effectively move milk toward the nipple of the breast for expression therefrom.

In embodiments, the controller and supporting electronics of PCB of the removable part of the right massage unit can be configured to supply electrical signals to the electric motor of the removable part to provide power and control of the electric motor in driving the mechanical massage actuation of the right breast as provided by cooperation of the motor, the transmission, and the massage actuator of the right massage unit. The controller and supporting electronics of the PCB of the removable part of the right massage unit can also be configured to supply electrical signals to the electric suction pump of the removable part to control the operation of the electric suction pump in supplying suction to the external suction head and bottle assembly via a suction port in the removable part. A suction line can be fluidly coupled to this suction port to connect to the external suction head and bottle assembly. In this configuration, the negative pressure (suction) generated by the electric suction pump of the removable part can be supplied to the external suction head and bottle assembly.

The removable part of the right massage unit can also support a wireless antenna that interfaces to the controller and supporting electronics of the PCB of the removable part. The controller and supporting electronics of the PCB can cooperate with the wireless antenna to provide a wireless communication link to a computing device. The wireless communication link can support a standardized wireless communication protocol such as any one of a number of Bluetooth^{®} protocols (e.g., Bluetooth^{®} v1.0 to v1.08, Bluetooth^{®} v1.1, Bluetooth^{®} v1.2, and Bluetooth^{®} v2.0), or any one of a number of Wi-Fi or IEEE 802.11 protocols, or other wireless data communication protocols. The computing device can be a smartphone, smartwatch, tablet computer, or another computing device. For example, FIG. 8 depicts a smartphone labeled 1105. Alternatively or additionally, the computing device can be a wireless remote device, which is labeled 1107 in FIG. 8. The computing device may execute software (e.g., an application, a.k.a., an "app") that graphically displays an operational interface to a user. The operational interface may be used to configure and/or control the operating parameters of the right massage unit, including operating parameters of the electric motor that drives the massage actuation of the right massage unit and operating parameters of the electric suction pump of the right massage unit. The operating parameters may be saved locally on the computing device or remotely (for example, in cloud data storage) during use for retrieval and/or analysis.

Furthermore, the removable part of the right massage unit can include a port for receiving DC electrical power that charges the one or more batteries of the removable part. When charged, the one or more batteries of the removable part can be used for supply of electrical power to the electrical components of the removable part of the right massage unit, including the controller and supporting electronics of the PCB, the electric motor, and the electric suction pump. Optionally, the DC electrical power can also provide electrical power to the electrical components of the removable part. The electrical power can be regulated for supply to the electrical components of the removable part as needed.

In embodiments, the right-wing panel 21b of the bra 1 can include a pocket or sleeve formed from fabric and similar to the pocket or sleeve 24a as shown in FIGS. 3 and 6. In embodiments, the fabric of the pocket or sleeve can be a jacquard knit that is knitted or sewn or otherwise joined to the right-wing panel 21b. The dimensions and material of the pocket or sleeve can be configured to enclose and secure at least part of the right massage unit 22b unit during use. For example, the dimensions and material of the pocket or sleeve in the right-wing panel 21b can be configured to retain and hold the removable part of the right massage unit. In this configuration, the bra 1 can support the removable part of the right massage unit in a position under the right arm of the user that is hidden (or at least partially hidden) from view during normal interaction with others, which enables the right massage unit to be worn and used in a discrete manner. In embodiments, an opening leading into the pocket or sleeve extends laterally across the top side of the pocket or sleeve similar to the opening 26a for the pocket or sleeve 24a for the left massage unit, while a smaller vertical opening leading into the pocket or sleeve extends along the front side of the pocket or sleeve similar to the opening 28a for the pocket or sleeve 24a for the left massage unit. The top side opening can be sized to accommodate the lengthwise dimension of the removable part of the right massage unit to enable the removable part to be placed into the pocket or sleeve or removed from the pocket or sleeve. The front-side opening can be sized to accommodate the height dimension of the on-pad part of the right massage unit such that the on-pad part extends through the front-side opening to a position at or near pad 3b.

### MASSAGE UNITS EMPLOYING SEQUENTIAL LINEAR PULL ACTION

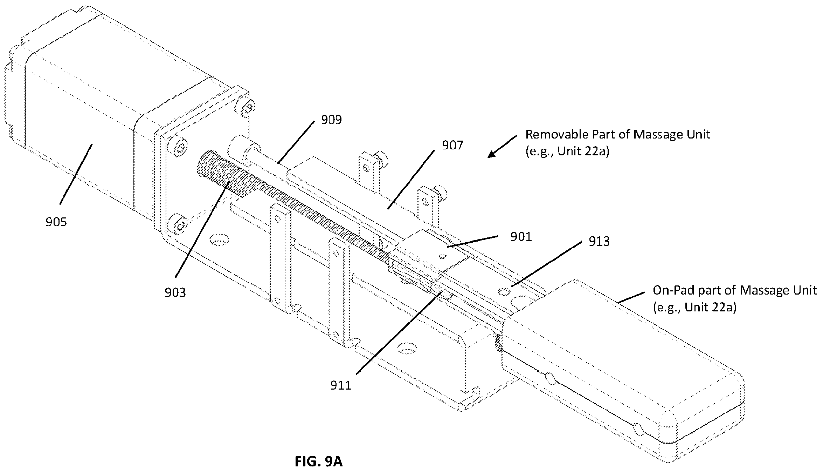

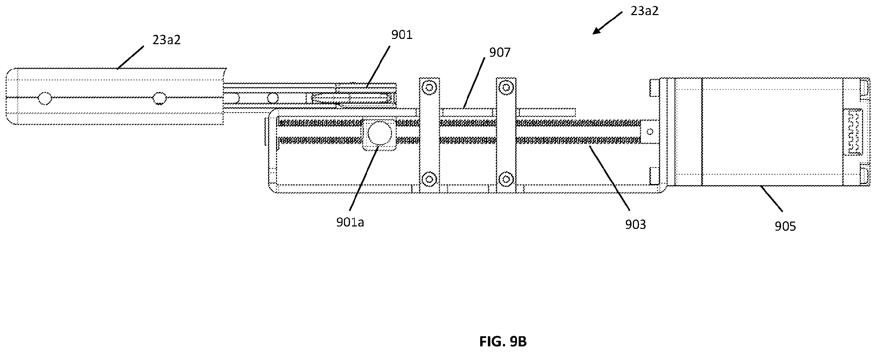

In one embodiment illustrated in FIGS. 9A to 9F, the removable part of a respective massage unit (e.g., the left massage unit 22a of FIGS. 7 and 8, or a similar right massage unit) can include a slider 901 that is mechanically coupled to a leadscrew 903 that is rotatably driven by an electric motor 905 of the removable part. The rotation of the leadscrew 903 is converted to linear motion of the slider 901 along a track 907 that extends longitudinally within the interior space of the removable part as shown. To accomplish such motion, the slider 901 includes a base 901a with a female threaded interface that receives and surrounds the leadscrew 902. The base 901a also includes a slip interface that receives and surrounds a fixed shaft 909 that extends parallel to and below the track 907 as best shown in FIG. 9A. The slip interface permits the slider 901 to slide or move linearly along the fixed shaft 909 and track 907 while prohibiting rotational movement of the slider 901 about the leadscrew 902. The slider 901 further supports a lock/unlock mechanism 911 that includes spring-biased arms whose distal ends selectively capture an end wall of a sequential pull mechanism 913 of the on-pad part of the massage unit. The spring-biased arms of the lock/unlock mechanism 911 can be pivoted (for example, automatically at a predefined home position of the slider 901 or by the user manually pressing finger tabs coupled to the arms) to mechanically decouple the slider 901 from the sequential pull mechanism 913 and permit disconnection of the removable part from the on-pad part. The removable part can be reconnected to the on-pad part such that the distal ends of the spring-biased arms capture the end wall of the sequential pull mechanism 913, which effectively mechanically couples the slider 901 to the sequential pull mechanism 913.

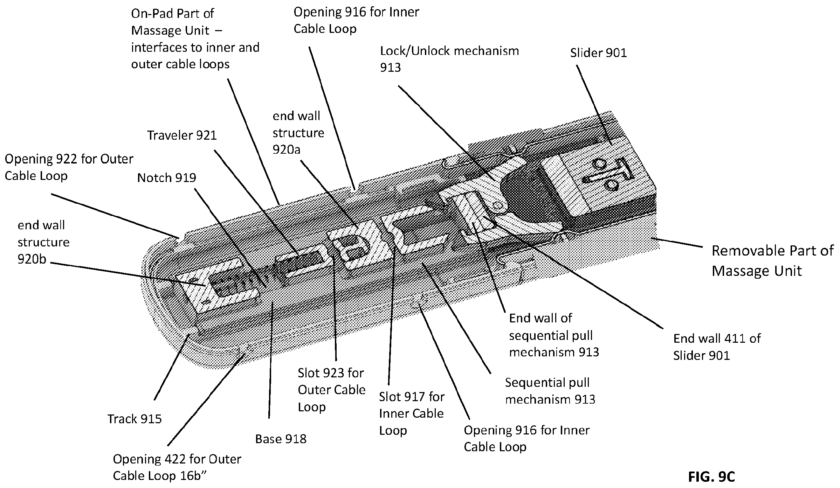

As best shown in FIGS. 9C to 9F, the sequential pull mechanism 913 is configured to slide linearly along a track 915 that extends longitudinally within the interior space of the on-pad part. The track 915 is aligned with track 907 of the removable part. In this configuration, when the removable part is connected to the on-pad part, the sequential pull mechanism 913 and the slider 901 can translate linearly along the track parts (915, 907) under actuation provided by leadscrew 903 of the removable part.

The on-pad part has opposed openings 916 that permit a lengthwise section of the inner cable loop to extend into and through the interior space of the on-pad part. The sequential pull mechanism 913 has opposed walls that form a slot 917 that engages and captures such length-wise section of the inner cable loop. A mechanical fastener (such as a screw) and/or adhesive can be used to secure the inner cable loop in slot 917.

The sequential pull mechanism 913 includes a base 918 that defines a notch 919 that extends parallel to the track 915 between opposed end wall structures 920a, 920b. A traveler 921 is positioned within the notch 919 and can move linearly within the notch 919 between the opposed end wall structures 920a, 920b under certain translation movement of the sequential pull mechanism 913.

The on-pad part also has opposed openings 922 that permit a lengthwise section of the outer cable loop to extend into and through the interior space of the on-pad part. The traveler 921 has opposed walls that form a slot 923 that engages and captures such a length-wise section of the outer cable loop. A mechanical fastener (such as a screw) and/or adhesive can be used to secure the outer cable loop in slot 923.

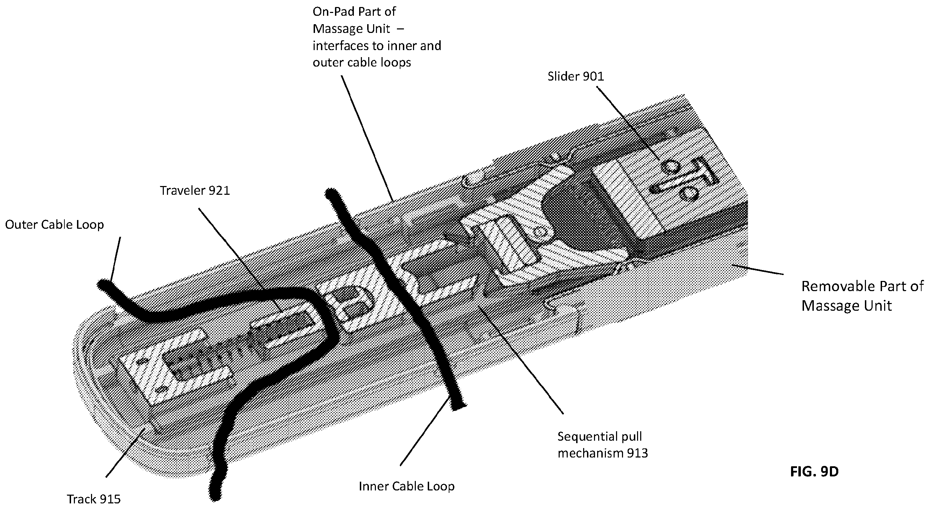

Figure 9D shows the relative positions of the sequential pull mechanism 913/slider 901 and the traveler 921 and the cable loops secured thereto in a neutral position in which tension is not applied to any of the cable loops, corresponding to a neutral position in which the breast is not being compressed. Whenever any one of the sequential pull mechanism 913/slider 901 and the traveler 921 moves linearly upward and to the right in Figure 9D away from its respective neutral position under actuation provided by the massage actuator (e.g., leadscrew 903), such movement selectively pulls or otherwise displaces the corresponding cable loop to decrease the effective length of the corresponding cable loop that surrounds the breast, which results in radial inward displacement of the corresponding pad (e.g., pad 3a or pad 3b) and compression of the breast of the user. Owing to the arrangement of the notch 919 and the opposed end wall structures 920a, 920b, the timing and sequence of the linear movement of the sequential pull mechanism 913/slider 901 and the traveler 921 and the resulting decrease in the effective lengths of the corresponding cable loops and application of tension to the cable loops and breast compression can be controlled during each stroke of the sequential pull mechanism 913/slider 901 upward and to the right is Figure 9D away from its respective neutral position under actuation provided by the massage actuator (e.g., leadscrew 903). This stroke corresponds to a compression phase of the compression-expansion cycle.

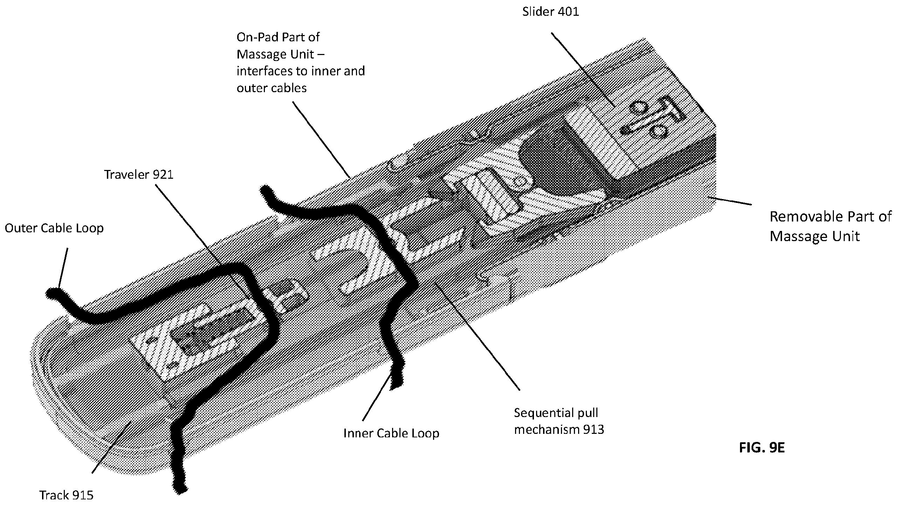

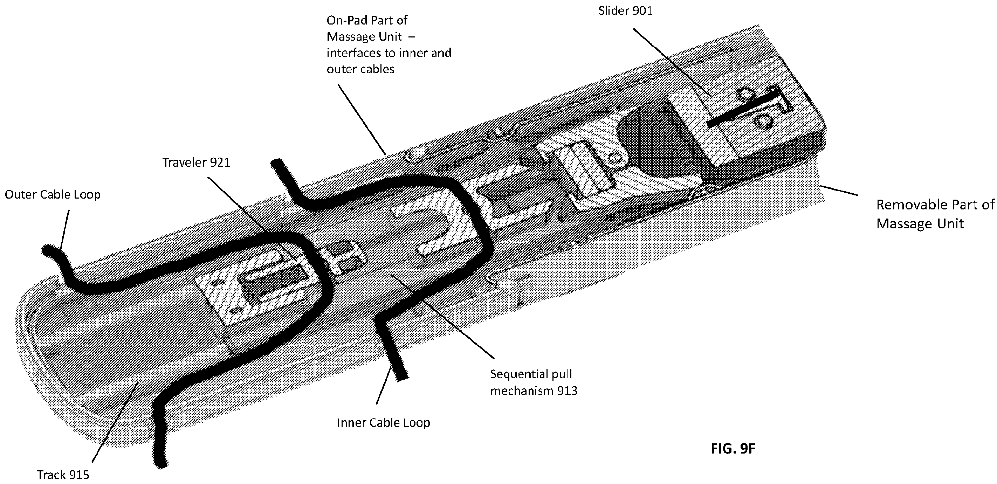

The operation of the massage unit may be understood with reference to FIGS. 9D, 9E, and 9F. As the slider 901 and sequential pull mechanism 913 initially move away from their respective neutral positions (Figure 9D) under actuation provided by the massage actuator (e.g., leadscrew 903), the sequential pull mechanism 913 will move along the track 915 without engaging and moving the traveler 921. In this configuration as shown in Figure 9E, the movement of the sequential pull mechanism 913 pulls on the inner cable loop to decrease the effective length of the inner cable loop that surrounds the breast, which results in radial inward displacement of the corresponding pad and compression of the breast of the user. The traveler 921 remains at or close to the neutral position and thus produces little or no displacement of the corresponding outer cable loop. The length of the inner cable loop that is pulled by the upward movement of sequential pull mechanism 913 and slider 901 is gathered within the interior space of the bra massage unit. Continued movement of the slider 901 and sequential pull mechanism 913 away from their respective neutral positions under actuation provided by the massage actuator (e.g., lead screw 903) will then cause the traveler 921 to move to a position where the traveler 921 is positioned at or near the distal end wall structure 920b as shown in Figure 9F. In this position, the traveler 921 moves upward with the sequential pull mechanism 913 and slider 901. Thereafter, continued movement of the slider 901 and sequential pull mechanism 193 away from their respective neutral positions under actuation provided by the massage actuator (e.g., lead screw 903) will cause the traveler 921 to move together with the slider 901 and sequential pull mechanism 913. In this configuration, the movement of the sequential pull mechanism 913 pulls on the inner cable loop to further decrease the effective length of the inner cable loop that surrounds the breast and the movement of the traveler 921 pulls on the outer cable loop to decrease the effective length of the outer cable loop that surrounds the breast, which results in radial inward displacement of the corresponding pad and further compression of the breast of the user. The length of the inner cable loop that is pulled by the continued upward movement of sequential pull mechanism 913 and slider 901 is gathered within the interior space of the massage unit, and the length of the outer cable loop that is pulled by the upward movement of traveler 921 is also gathered within the interior space of the massage unit. Such compression continues until the slider 901 and sequential pull mechanism 913 and traveler 921 stops moving, which ends the compression stroke. Thereafter, the massage unit may be configured to begin an expansion stroke of the slider 901 and sequential pull mechanism 913 and traveler 921 and an expansion phase of the compression-expansion cycle, which increase the effective lengths of both the inner and outer cable loops that surround the breast to remove tension in such cable loops, allowing the slider 901 and sequential pull mechanism 913 and traveler 921 and the cable loops secured thereto to move downward and return back to their neutral positions shown in Figure 9C, completing the compression-expansion cycle. The compression-expansion cycle can be repeated multiple times to facilitate breast milk expression. Thus, based on the compression sequence, the inner cable loop will compress the breast first, followed by compression of the breast by the outer cable loop, such that the breast will be compressed progressively in an outward direction (e.g., longitudinal direction A in FIG. 6) from the base of the breast toward the nipple area of the breast. Also, it will be appreciated that during each compression stroke, the inner cable loop will be in tension for a longer period of time than the outer cable loop.

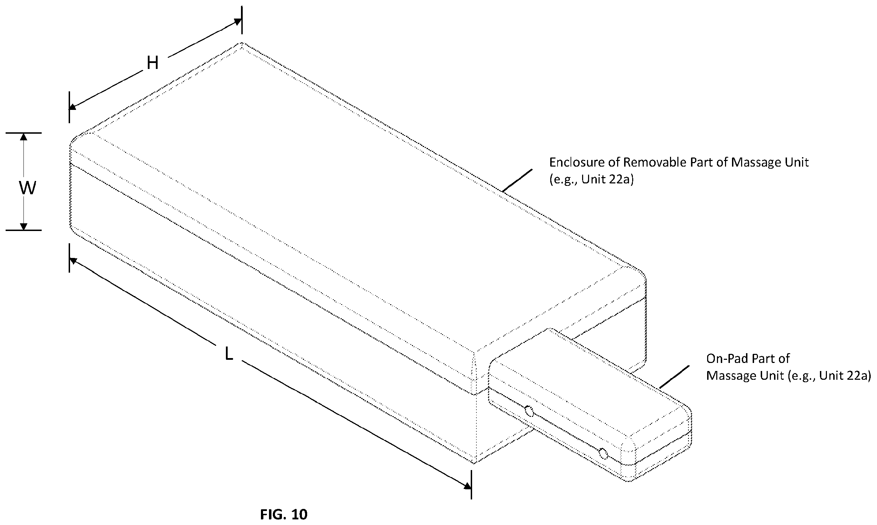

In embodiments, the removable part of each respective left and right massage unit can employ an elongate rectangular enclosure with characteristic length (L), height (H), and width (W) dimensions as shown in FIG. 10. The pocket or sleeve (24a or 24b) of the bra can be configured with length and height dimensions that are greater than (or equal) to the corresponding length (L) and height (H) dimensions of the enclosure of the removable part of the massage unit. The elasticity of the fabric of the pocket or sleeve (24a or 24b) can be configured to accommodate the width (W) dimension of the enclosure of the removable part of the massage unit. Note that with the respective removable part of the massage unit stowed in the corresponding pocket or sleeve (24a or 24b) of the bra, the length dimension (L) of the enclosure and the corresponding length dimension of the pocket or sleeve (24a or 24b) generally extend parallel to the Anterior-Posterior direction of the body of the user, the height dimension (H) of the enclosure and the corresponding width dimension of the pocket or sleeve (24a or 24b) generally extend parallel to the Superior-Inferior direction of the body of the user, and the width dimension (W) of the enclosure generally extends parallel to the Medial-Lateral direction of the body of the user. In embodiments, the characteristic length (L) dimension of the enclosure can be in the range of 130-170mm, the characteristic height (H) dimension of the enclosure can be in the range of 65-75mm, and the characteristic width (W) dimension of the enclosure can be in the range of 25-40mm. In one exemplary embodiment, the characteristic length (L) dimension can be at or near 158.75mm, the characteristic height (H) dimension can be at one near 70.5mm, and the characteristic width (W) dimension can be at or near 30mm. These dimensional ranges provide a low-profile design that can be worn comfortably under the arm of the user during use.

### MASSAGE UNITS EMPLOYING SEQUENTIAL ROTATIONAL PULL ACTION

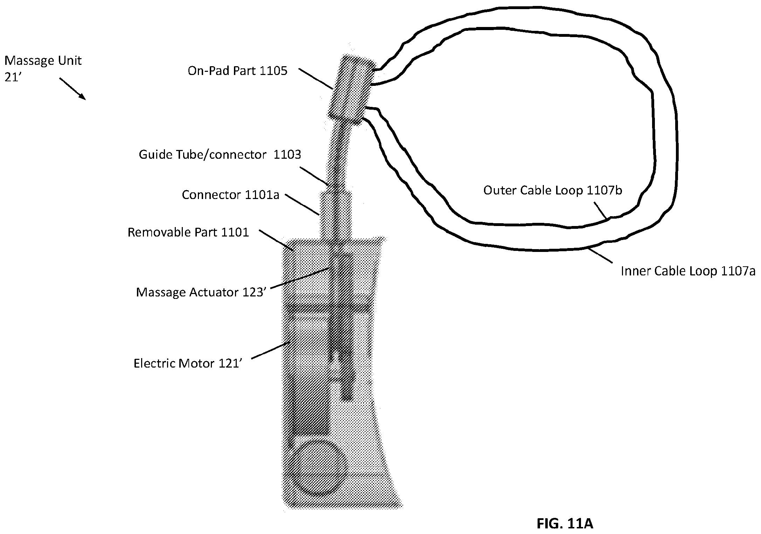

In other embodiments, the bra 1 can support a massage unit 21' for massage of the left breast or the right breast, where the massage unit 21' includes a guide tube/connector 1103 that is operably coupled between a removable part 1101 and an on-pad part 1105 as illustrated in FIGS. 11A to 11D. The on-pad part 1105 and guide tube 1103 are rigidly secured to one another and the guide tube 1103 is curved to guide an inner cable loop 1107a (or part thereof) and outer cable loop 1107b (or part thereof) to the removable part 1101. The cable loops 1107a, 1107b extend through the channels 17a1, 17a2, respectively of pad 3a for the left breast massage unit, or through channels 17b1, 17b2, respectively of pad 3b for the right breast massage unit as described herein.

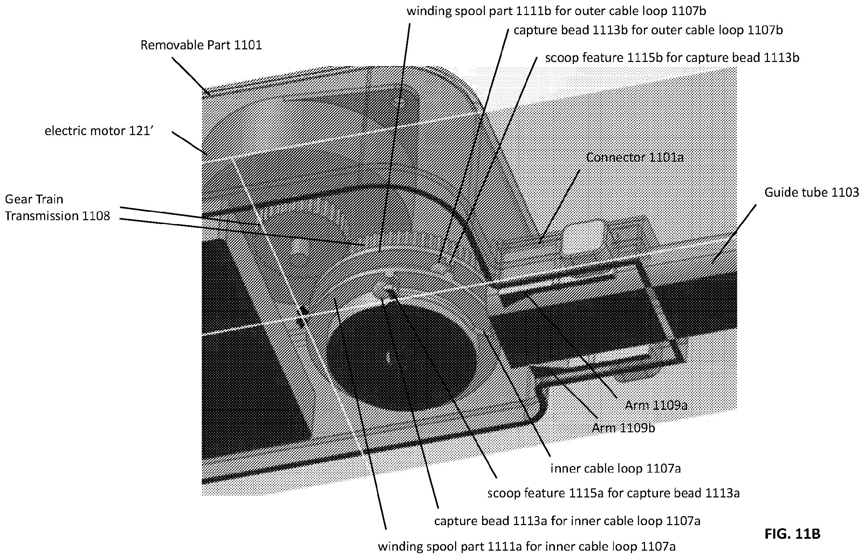

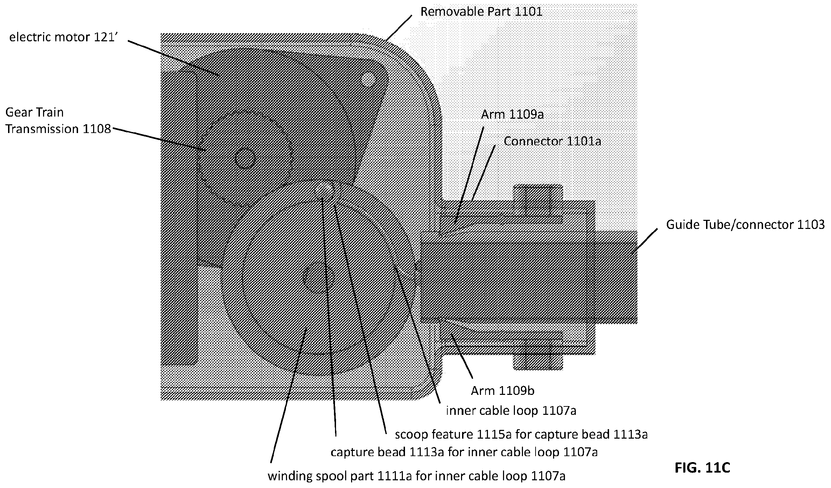

In the embodiment shown in FIGS. 11B and 11C, the removable part 1101 includes a connector 1101a that supports opposed arms 1109a, 1109b whose distal ends selectively capture slots or grooves at or near the end of the guide tube/connector 1103 as shown. The arms 1109a, 1109b can be pivoted by the user manually pressing finger tabs coupled to the arms to mechanically decouple the removable part 1101 from the guide tube/connector 1103 and permit disconnection of the removable part 1101 from the guide tube/connector 1103 and the on-pad part 1105. The removable part 1101 can be reconnected to the guide tube/connector 1103 and on-pad part 1105 such that the distal ends of the arms 1109a, 1109b capture the slots or grooves at or near the end of the guide tube/connector 1103, which effectively mechanically couples the removable part 1101 to the guide tube/connector 1103 and the on-pad part 1105. In other embodiments, other mechanical elements can be used to connect and reconnect the removable part 1101 to the guide tube/connector 1103 and on-pad part 1105.

Similar to the embodiment of FIGS. 7 and 8, the removable part 1101 of the massage unit 21' can house a PCB with a controller and supporting electronics, electric motor 121' (e.g., stepper motor), at least one battery (e.g., lithium-ion battery), and a massage actuator 123' (e.g., gear train transmission and rotary pull mechanism for the cable loops). The operation of the massage actuator 123' is driven by the rotary output of the electric motor 121'.

In embodiments, the electric motor 121' and massage actuator 123' can cooperate to apply one or more compression-expansion cycles to the cable loops 1107a, 1107b. Each compression-expansion cycle selectively displaces the cable loops to decrease the effective lengths of the cable loops that surround the breast to compress the breast, and then displaces the cable loops to increase the effective lengths of the cable loops that surround the breast to permit the breast to expand and decompress. In embodiments, each compression-expansion cycle includes compression operations that sequentially displace the cable loops to decrease the effective lengths of the cable loops that surround the breast from the inner cable loop (closer to the torso) to the outer cable (furthest from the torso) without removal of tension from the cable loops in order to apply sequential compression to the breast. Each compression-expansion cycle further includes expansion operations that displace the cable loops to increase the effective lengths of the cable loops that surround the breast to permit expansion of the breast. The sequential compression operations followed by the expansion operations as part of the compression-expansion cycle mimics massaging of the breast and can effectively move milk toward the nipple of the breast for expression therefrom.

In embodiments, the controller and supporting electronics of PCB of the removable part 1101 can be configured to supply electrical signals to the electric motor 121' of the removable part 1101 to provide power and control of the electric motor 121' in driving the mechanical massage actuation of the breast as provided by cooperation of the motor 121' and the massage actuator 123' of the massage unit. The controller and supporting electronics of the PCB of the removable part 1101 can also be configured to supply electrical signals to the electric suction pump of the removable part 1101 to control the operation of the electric suction pump in supplying suction to the external suction head and bottle assembly via a suction port in the removable part. A suction line can be fluidly coupled to this suction port to connect to the external suction head and bottle assembly. In this configuration, the negative pressure (suction) generated by the electric suction pump of the removable part can be supplied to the external suction head and bottle assembly.

The removable part 1101 of the massage unit 21' can also support a wireless antenna that interfaces to the controller and supporting electronics of the PCB of the removable part. The controller and supporting electronics of the PCB can cooperate with the wireless antenna to provide a wireless communication link to a computing device. The wireless communication link can support a standardized wireless communication protocol such as any one of a number of Bluetooth^{®} protocols (e.g., Bluetooth^{®} v1.0 to v1.08, Bluetooth^{®} v1.1, Bluetooth^{®} v1.2, and Bluetooth^{®} v2.0), or any one of a number of Wi-Fi or IEEE 802.11 protocols, or other wireless data communication protocols. The computing device can be a smartphone, smartwatch, tablet computer, or another computing device. For example, FIG. 8 depicts a smartphone labeled 1105. Alternatively or additionally, the computing device can be a wireless remote device, which is labeled 1107 in FIG. 8. The computing device may execute software (e.g., an application, a.k.a., an "app") that graphically displays an operational interface to a user. The operational interface may be used to configure and/or control the operating parameters of the massage unit 21', including operating parameters of the electric motor that drives the massage actuation of the massage unit 21' and operating parameters of the electric suction pump of the massage unit 21'. The operating parameters may be saved locally on the computing device or remotely (for example, in cloud data storage) during use for retrieval and/or analysis.

Furthermore, the removable part 1101 can include a port for receiving DC electrical power that charges the one or more batteries of the removable part 1101. When charged, the one or more batteries of the removable part 1101 can be used for supply of electrical power to the electrical components of the removable part 1101, including the controller and supporting electronics of the PCB, the electric motor 121', and the electric suction pump. Optionally, the DC electrical power can also provide electrical power to the electrical components of the removable part 1101. The electrical power can be regulated for supply to the electrical components of the removable part 1101 as needed.

In embodiments, the left-wing panel 21a and the right-wing panel 21b of the bra 1 (FIGS. 1 to 6) can each include a pocket or sleeve formed from fabric and similar to the pocket or sleeve 24a as shown in FIGS. 3 and 6. The dimensions and material of the pocket or sleeve in the respective left wing panel 21a and right-wing panel 21b can be configured to retain and hold the removable part 1101 of the massage unit 21'. In this configuration, the bra 1 can support the removable part 1101 of the massage unit in a position under the arm of the user that is hidden (or at least partially hidden) from view during normal interaction with others, which enables the massage unit to be worn and used in a discrete manner. In embodiments, an opening leading into the pocket or sleeve extends laterally across the top side of the pocket or sleeve similar to the opening 26a for the pocket or sleeve 24a, while a smaller vertical opening leading into the pocket or sleeve extends along the front side of the pocket or sleeve similar to the opening 28a for the pocket or sleeve 24a. The top side opening can be sized to accommodate the lengthwise dimension of the removable part 1901 of the massage unit to enable the removable part to be placed into the pocket or sleeve or removed from the pocket or sleeve. The front-side opening can be sized to accommodate the dimensions of the on-pad part 1105 and/or the guide tube 1103 such that the guide tube 1103 extends through the front-side opening with the on pad-part 1105 disposed at a position at or near the corresponding pad (3a or 3b).

In the embodiment of FIGS. 11B to 11D, the massage actuator 123' of the removable part 1101 includes a gear train transmission 1108 driven by the output shaft of the electric motor 121'. The rotational output of the gear train transmission 1108 drives a rotational pull mechanism that includes a two-part winding spool, which has one part 1111a for the inner cable loop 1107a and another part 1111b for the outer cable loop 1107b. The inner cable loop 1107a is terminated by capture bead 1113a, and the outer cable loop 1107b is terminated by capture bead 1113b. The winding spool part 1111a includes a scoop feature 1115a that captures and engages the capture bead 1113a of the inner cable loop 1107a during certain rotation of the winding spool (in the counterclockwise sense away from the guide tube/connector 1103). The scoop feature 1115a has a slot that permits the inner cable loop 1107a to extend through the slot when the capture bead 1113a is engaged by the scoop feature 1115a. With the capture bead 1113a engaged by the scoop feature 1115a, further rotation of the winding spool (in the counterclockwise sense away from the guide tube/connector 1103) causes the inner cable loop 1107a to wind around the winding spool part 1111a and decreases the effective length of the inner cable loop 1107a. Similarly, the winding spool part 1111b includes a scoop feature 1115b that captures and engages the capture bead 1113b of the outer cable loop 1107b during certain rotation of the winding spool (in the counterclockwise sense away from the guide tube/connector 1103). The scoop feature 1115b has a slot that permits the outer cable loop 1107b to extend through the slot when the capture bead 1113b is engaged by the scoop feature 1115b. With the capture bead 1113b engaged by the scoop feature 1115b, further rotation of the winding spool (in the counterclockwise sense away from the guide tube/connector 1103) causes the outer cable loop 1107b to wind around the winding spool part 1111b and decreases the effective length of the outer cable loop 1107b. In order to accommodate sequential pulling of the outer cable loop 1107b after the inner cable loop 1107a as described herein, the scoop feature 1115a can be offset from the scoop feature 1115b by a predefined lead angle of rotation of the winder spool (in the counterclockwise sense of rotation of the winding spool parts) as shown.

When the connector 1101a is connected (or reconnected) to the end of the guide tube/connector 1103 as best shown in FIGS. 11B and 11C, the capture beads 1113a, 1113b can be positioned in a neutral position adjacent the outlet of the guide tube/connector 1103 within the respective winding spool parts 1111a, 1111b. In this neutral position, no tension (or little tension) is applied to any of the cable loops and the breast is not being compressed. When the winding spool parts 1111a, 1111b rotate counterclockwise away from the guide tube/connector 1103 under actuation provided by the electric motor 121' and gear train transmission 1108, such movement causes the scoop features 1115a, 1115b to selectively capture and wind the corresponding cable loops 1107a, 1107b about the winding spool parts 1111a, 1111b to decrease the effective length of the corresponding cable loops that surrounds the breast, which results in radial inward displacement of the corresponding pad (e.g., pad 3a or pad 3b) and compression of the breast of the user. Owing to the arrangement of the angular offset between the scoop feature 1115a and scoop feature 1115b, the timing and sequence of the rotational capture and winding of the corresponding cable loops 1107a, 1107b and the resulting decrease in the effective lengths of the corresponding cable loops and application of tension to the cable loops and breast compression can be controlled during each rotational stroke of the winder spool away from the neutral position of the capture beads 1113a, 1113b under rotational actuation provided by the electric motor 121' and the gear train transmission 1108. This stroke corresponds to the compression phase of the compression-expansion cycle.

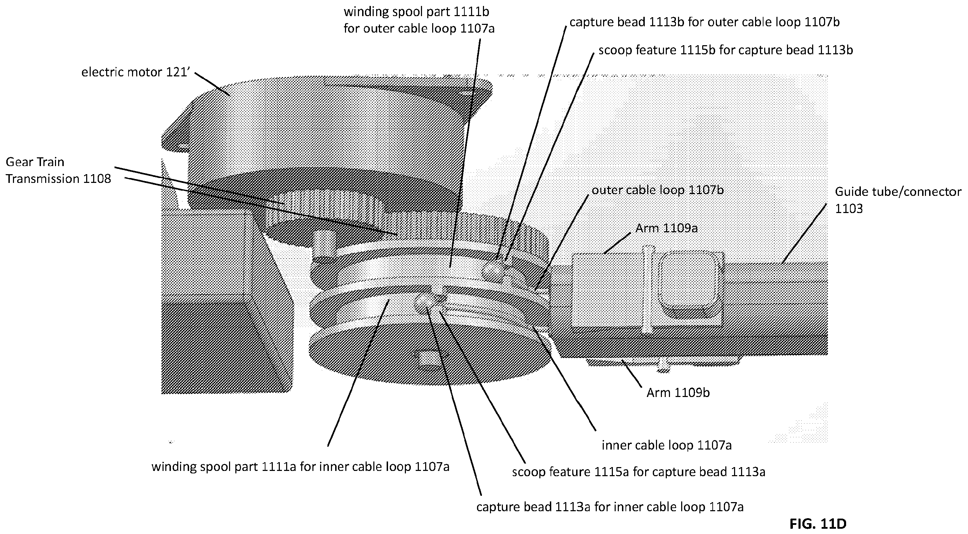

The operation of the massage unit 21' may be understood with reference to FIG. 11D. When the capture beads 1113a, 1113b are positioned in their neutral position and the winding spool parts 1111a, 1111b rotate counterclockwise away from the guide tube/connector 1103 under actuation provided by the electric motor 121' and the gear train transmission 1108, such movement causes the scoop feature 1115a to capture the capture bead 1113a and wind the inner cable loop 1107a about the winding spool part 1111a (without the scoop feature 1115b capturing the capture bead 1113b and winding the outer cable loop 1107b) to decrease the effective length of the inner cable loop 1107a that surrounds the breast, which results in radial inward displacement of the corresponding pad (e.g., pad 3a or pad 3b) and compression of the breast of the user. The capture bead 1113b remains at or close to its neutral position and thus produces little or no displacement of the corresponding outer cable loop 1107b. The length of the inner cable loop 1107a that is pulled by the rotational movement of the winding spool parts 1111a, 1111b is gathered by the winding spool part 1111a within the interior space of the massage unit 21'. Continued rotational movement of the winding spool parts 1111a, 1111b counterclockwise away from the guide tube/connector 1103 under rotational actuation provided by the electric motor 121' and the gear train transmission 1108 causes the scoop feature 1115b to capture the capture bead 1113b and wind the outer cable loop 1107b about the winding spool part 1111b (with the scoop feature 1115a further engaging the capture bead 1113a and winding the inner cable loop 1107a) to decrease the effective length of both the inner cable loop 1107a and outer cable loop 1107b. This results in radial inward displacement of the corresponding pad (e.g., pad 3a or pad 3b) and compression of the breast of the user. The length of the outer cable loop 1107b that is pulled by the rotational movement of the winding spool parts 1111a, 1111b is gathered by the winding spool part 1111b within the interior space of the massage unit 21'. Thereafter, continued rotational movement of the winding spool parts 1111a, 1111b counterclockwise away from the guide tube/connector 1103 under rotational actuation provided by the electric motor 121' and the gear train transmission 1108 causes the scoop feature 1115a to further engage the capture bead 1113a and wind the inner cable loop 1107a about the winding spool part 1111a and the scoop feature 1115b to further engage the capture bead 1113b and wind the outer cable loop 1107b about the winding spool part 1111b, which decreases the effective length of both the inner cable loop 1107a and outer cable loop 1107b. This results in further radial inward displacement of the corresponding pad (e.g., pad 3a or pad 3b) and compression of the breast of the user. Such compression continues until the counterclockwise rotational movement of the winding spool parts 1111a, 1111b stops, which ends the compression stroke. Thereafter, the massage unit 21' may be configured to begin an expansion stroke of the winding spool parts 1111a, 1111b and an expansion phase of the compression-expansion cycle, which increase the effective lengths of both the inner and outer cable loops 1107a, 1107b that surround the breast to remove tension in such cable loops, by rotating the scoop features 1115a, 1115b clockwise and returning the capture beads 1113a, 1113b back to their neutral positions, completing the compression-expansion cycle. The compression-expansion cycle can be repeated multiple times to facilitate breast milk expression. Thus, based on the compression sequence, the inner cable loop 1107a will compress the breast first, followed by compression of the breast by the outer cable loop 1107b, such that the breast will be compressed progressively in an outward direction (e.g., longitudinal direction A in FIG. 6) from the base of the breast toward the nipple area of the breast. Also, it will be appreciated that during each compression stroke, the inner cable loop 1107a will be in tension for a longer period of time than the outer cable loop 1107b.

In other embodiments, the configuration of the scoop features of the winding spool of the massage actuator and/or the rotational speed of the electric motor 121' can be readily adapted to provide variable delays in the peak of the compression cycle or variable speeds of compression/decompression cycles.

In other embodiments, the massage actuator of the removable part 1101 can possibly omit the gear train transmission. In this embodiment, the rotational torque provided by the output shaft of the electric motor 121' can be directly coupled to the winding spool and not require the mechanical advantage of the gear train transmission.

In still other embodiments, the capture beads and corresponding scoop features of the rotary winding spool of the massage actuator of the removable part 1101 can be replaced with other mechanical structures that detachably couple the cable loops to the winding spool with delayed winding of the outer cable relative to the inner cable for sequential tensioning of the inner and outer cables as described herein.

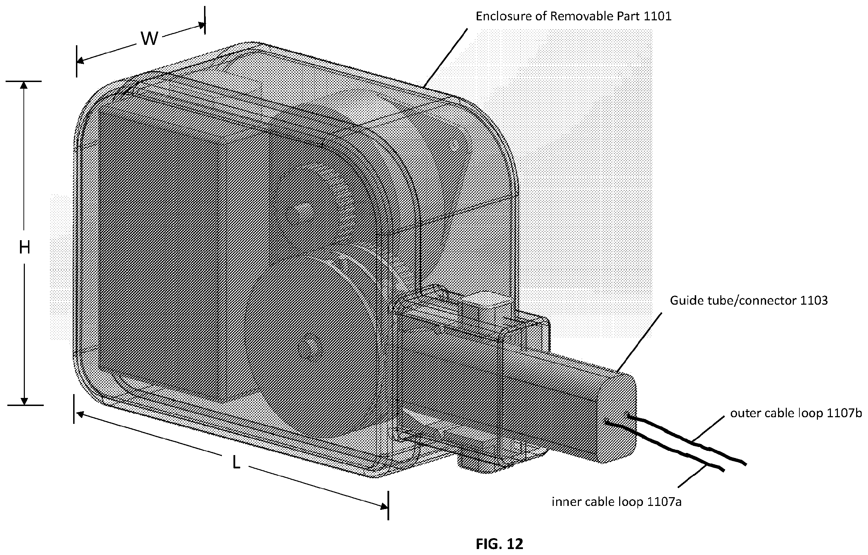

In embodiments, the removable part 1101 of the massage unit 21' can employ an elongate rectangular enclosure with characteristic length (L), height (H) and width (W) dimensions as shown in FIG. 12. The pocket or sleeve (24a or 24b) of the bra can be configured with length and width dimensions that are greater than (or equal) to the corresponding length (L) and width (W) dimensions of the enclosure of the removable part 1101 of the massage unit 21'. The elasticity of the fabric of the pocket or sleeve (24a or 24b) can be configured to accommodate the height (H) dimension of the enclosure of the removable part 1101 of the massage unit 21'. Note that with the removable part 1101 of the massage unit 21' stowed in the corresponding pocket or sleeve (24a or 24b) of the bra, the length dimension (L) of the enclosure and the corresponding length dimension of the pocket or sleeve (24a or 24b) generally extend parallel to the Anterior-Posterior direction of the body of the user, the height dimension (H) of the enclosure and the corresponding width dimension of the pocket or sleeve (24a or 24b) generally extend parallel to the Superior-Inferior direction of the body of the user, and the width dimension (W) of the enclosure generally extends parallel to the Medial-Lateral direction of the body of the user. In one embodiment, the characteristic length (L) dimension of the enclosure can be in the range of 4.0-5.0inches, the characteristic height (H) dimension of the enclosure can be in the range of 3.0-4.0inches, and the characteristic width (W) dimension of the enclosure can be in the range of 2.25-4.0inches. In another exemplary embodiment, the characteristic length (L) dimension of the enclosure can be in the range of 3.75-4.25inches, the characteristic height (H) dimension of the enclosure can be in the range of 2.5-3.0inches, and the characteristic width (W) dimension of the enclosure can be in the range of 2.0-2.25inches. In yet another exemplary embodiment, the characteristic length (L) dimension of the enclosure can be in the range of 3.0-3.75inches, the characteristic height (H) dimension of the enclosure can be in the range of 2.25-2.5inches, and the characteristic width (W) dimension of the enclosure can be in the range of 1.75-2.0inches. These dimensional ranges provide a low-profile design that can be worn comfortably under the arm of the user during use.

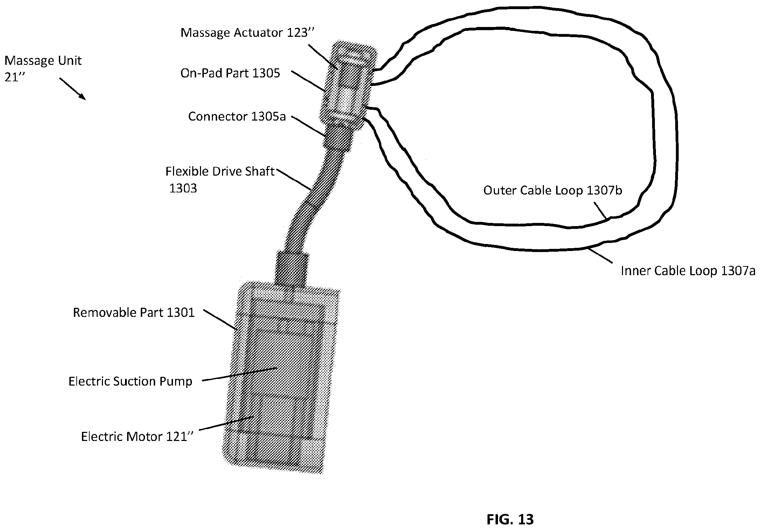

In another embodiment illustrated in FIG. 13, the bra 1 can support a massage unit 21" for massage of the left breast or the right breast, where the massage unit 21" includes a flexible drive shaft 1303 that is operably coupled between a removable part 1131 and an on-pad part 1305. The flexible drive shaft 1303 is designed to transmit rotary motion through a curved or curvilinear path. The removable part 1301 and flexible drive shaft 1303 are rigidly secured to one another and function to apply rotating shaft motion that actuates the massage actuator 123" of the on pad part 1305. The on-pad part 1305 interfaces to an inner cable loop 1307a and outer cable loop 1307b. The cable loops 1307a, 1307b extend through the channels 17a1, 17a2, respectively of pad 3a for the left breast massage unit, or through channels 17b1, 17b2, respectively of pad 3b for the right breast massage unit as described herein.

The on-pad part 1305 includes a connector 1305a that provides for connection and disconnection between the on-pad part 1305 and the flexible drive shaft 1303 (and the removable part 1301 coupled thereto). For example, the connector can include capture arms similar to those used for the connector 1101a described above.

Similar to the embodiment of FIGS. 7 and 8, the removable part 1101 of the massage unit 21" can house a PCB with a controller and supporting electronics, electric motor 1217" (e.g., stepper motor), and at least one battery (e.g., lithium-ion battery). The on-pad part 1105 can employ a massage actuator 123" (e.g., a gear train transmission and rotary pull mechanism for the cable loops). The operation of the massage actuator 123' is driven by the rotary output of the electric motor 121" transmitted thereto by the flexible drive shaft 1303.

In embodiments, the electric motor 121" and flexible drive shaft 1303 can cooperate with the massage actuator 123" of the on-pad part 1305 to apply one or more compression-expansion cycles to the cable loops 1307a, 1307b. Each compression-expansion cycle selectively displaces the cable loops to decrease the effective lengths of the cable loops that surround the breast to compress the breast, and then displaces the cable loops to increase the effective lengths of the cable loops that surround the breast to permit the breast to expand and decompress. In embodiments, each compression-expansion cycle includes compression operations that sequentially displace the cable loops to decrease the effective lengths of the cable loops that surround the breast from the inner cable loop (closer to the torso) to the outer cable (furthest from the torso) without removal of tension from the cable loops in order to apply sequential compression to the breast. Each compression-expansion cycle further includes expansion operations that displace the cable loops to increase the effective lengths of the cable loops that surround the breast to permit expansion of the breast. The sequential compression operations followed by the expansion operations as part of the compression-expansion cycle mimics massaging of the breast and can effectively move milk toward the nipple of the breast for expression therefrom.

In embodiments, the controller and supporting electronics of PCB of the removable part 1301 can be configured to supply electrical signals to the electric motor 121" of the removable part 1201 to provide power and control of the electric motor 121" in driving the mechanical massage actuation of the breast as provided by cooperation of the motor 121" and flexible drive shaft 1303 and the massage actuator 123" of the on-pad part 1305. The controller and supporting electronics of the PCB of the removable part 1301 can also be configured to supply electrical signals to the electric suction pump of the removable part 1301 to control the operation of the electric suction pump in supplying suction to the external suction head and bottle assembly via a suction port in the removable part. A suction line can be fluidly coupled to this suction port to connect to the external suction head and bottle assembly. In this configuration, the negative pressure (suction) generated by the electric suction pump of the removable part can be supplied to the external suction head and bottle assembly.

The removable part 1301 of the massage unit 21" can also support a wireless antenna that interfaces to the controller and supporting electronics of the PCB of the removable part. The controller and supporting electronics of the PCB can cooperate with the wireless antenna to provide a wireless communication link to a computing device. The wireless communication link can support a standardized wireless communication protocol such as any one of a number of Bluetooth^{®} protocols (e.g., Bluetooth^{®} v1.0 to v1.08, Bluetooth^{®} v1.1, Bluetooth^{®} v1.2, and Bluetooth^{®} v2.0), or any one of a number of Wi-Fi or IEEE 802.11 protocols, or other wireless data communication protocols. The computing device can be a smartphone, smartwatch, tablet computer, or another computing device. For example, FIG. 8 depicts a smartphone labeled 1105. Alternatively or additionally, the computing device can be a wireless remote device, which is labeled 1107 in FIG. 8. The computing device may execute software (e.g., an application, a.k.a., an "app") that graphically displays an operational interface to a user. The operational interface may be used to configure and/or control the operating parameters of the massage unit 21", including operating parameters of the electric motor that drives the massage actuation of the massage unit 21" and operating parameters of the electric suction pump of the massage unit 21". The operating parameters may be saved locally on the computing device or remotely (for example, in cloud data storage) during use for retrieval and/or analysis.

Furthermore, the removable part 1301 can include a port for receiving DC electrical power that charges the one or more batteries of the removable part 1301. When charged, the one or more batteries of the removable part 1301 can be used for supply of electrical power to the electrical components of the removable part 1301, including the controller and supporting electronics of the PCB, the electric motor 121", and the electric suction pump. Optionally, the DC electrical power can also provide electrical power to the electrical components of the removable part 1301. The electrical power can be regulated for supply to the electrical components of the removable part 1301 as needed.

In embodiments, the left-wing panel 21a and the right-wing panel 21b of the bra 1 (FIGS. 1 to 6) can each include a pocket or sleeve formed from fabric and similar to the pocket or sleeve 24a as shown in FIGS. 3 and 6. The dimensions and material of the pocket or sleeve in the respective left-wing panel 21a and right-wing panel 21b can be configured to retain and hold the removable part 1301 of the massage unit 21". In this configuration, the bra 1 can support the removable part 1301 of the massage unit in a position under the arm of the user that is hidden (or at least partially hidden) from view during normal interaction with others, which enables the massage unit to be worn and used in a discrete manner. In embodiments, an opening leading into the pocket or sleeve extends laterally across the top side of the pocket or sleeve similar to the opening 26a for the pocket or sleeve 24a, while a smaller vertical opening leading into the pocket or sleeve extends along the front side of the pocket or sleeve similar to the opening 28a for the pocket or sleeve 24a. The top side opening can be sized to accommodate the lengthwise dimension of the removable part 1301 of the massage unit to enable the removable part to be placed into the pocket or sleeve or removed from the pocket or sleeve. The front-side opening can be sized to accommodate the dimensions of the on-pad part 1305 and/or the flexible drive shaft 1303 such that the flexible drive shaft 1305 extends through the front-side opening with the on pad-part 1305 disposed at a position at or near the corresponding pad (3a or 3b).

In embodiments, the massage actuator 123" of the on-pad part 1305 can include a gear-like transmission driven by the flexible drive shaft 1303 and a spool-based rotational pull mechanism similar to the mechanism described above with respect to FIGS. 11B to 11D. This rotational pull mechanism can accommodate sequential pulling of the outer cable loop 1307b after the inner cable loop 1307a as described herein. In this embodiment, the winding spools of the on-pad part 1305 can be configured to rotate about a common rotational axis with a gear-like transmission disposed between the winding spools. The gear-like transmission interfaces to both the "outer" winding spool (that winds to the outer cable loop 1307b furthest from the torso) and the "inner" winding spool (that winds the inner cable loop 1307a nearest to the torso) and is configured such that the outer winding spool is not engaged by the gear-like transmission (and thus is not driven to rotate and wind the outer cable loop 1307b until the inner winder spool has rotated over a predefined amount or angle of rotation to provide the sequential pulling of the outer cable loop 1307b after the inner cable loop 1307a as described herein. In this embodiment, mechanical structures can be used to detachably couple the inner cable loop 1307a and the outer cable loop 1307b to the corresponding winding spools.

In embodiments, the system can include separate and distinct massage units that correspond to the left and right breasts of the user. While two massage units are shown, they may operate independently or together (e.g., simultaneously). Also, in other embodiments of the system, only one massage unit may be included, and a user may alternate its use from one breast to the other as desired. The massage unit(s) can be configured to apply compression to the tissue of the corresponding breast as described herein. The system can be used before, during, and/or after breast pumping or nursing to facilitate lactation and improve milk production and flow.

### MASSAGE UNIT EMPLOYING SEQUENTIAL ROTATIONAL PULL ACTION FOR LEFT AND RIGHT BREAST PADS

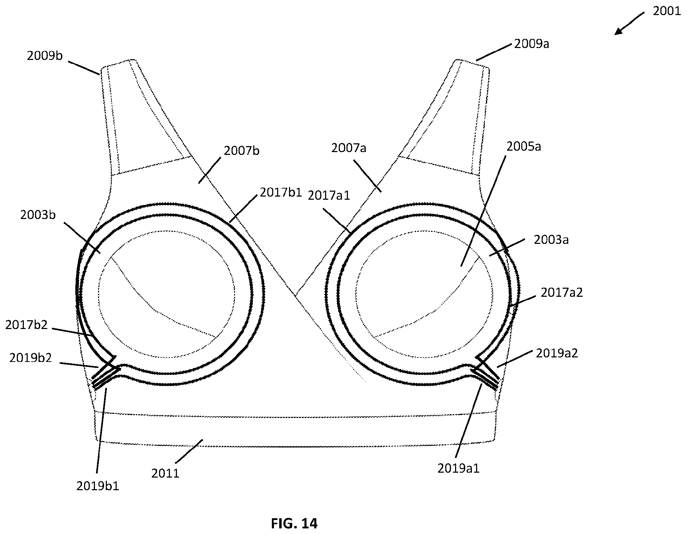

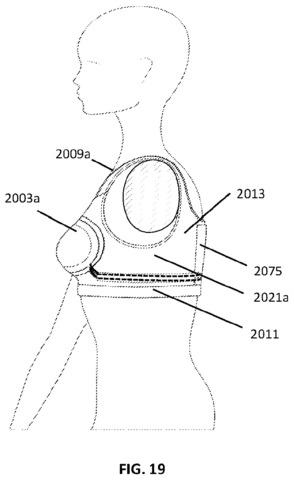

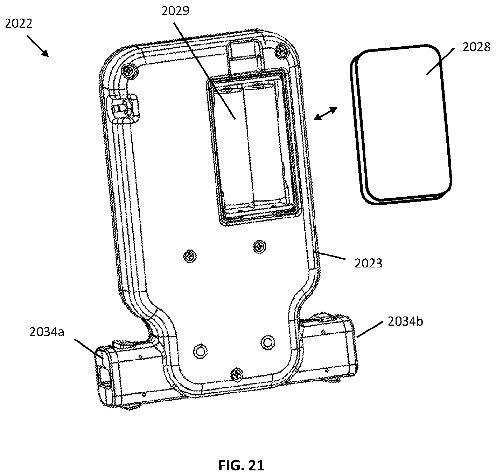

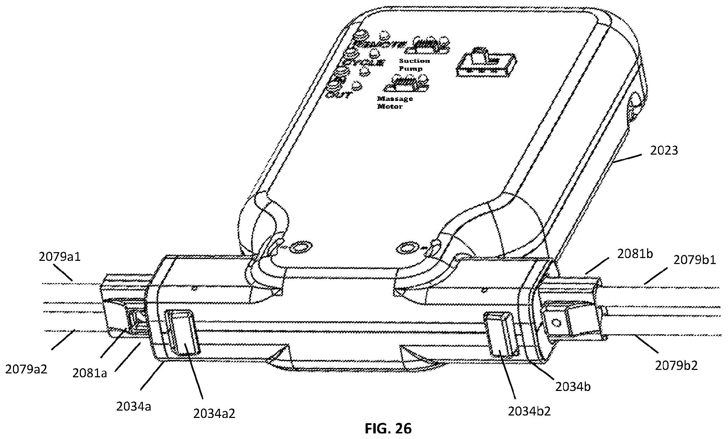

FIGS. 14 to 29 show another embodiment of a system that includes a single massage unit that removably supported by a bra 2001.

The bra 2001 includes a pad 2003a that is contoured and configured to surround and engage the left breast of a user during use. For purposes herein, the term "pad" is to be understood broadly to include one or pads or cups or shells that is contoured and configured to surround and engage a user's breast. The pad 2003a may be made from fabric, polymer, or any flexible material. The pad 2003a defines an opening or slit 2005a corresponding to the nipple area of the left breast. The opening or slit 2005a can be configured to receive therethrough a portion of a breast shield (not shown). The opening or slit 2005a can be defined by overlapping fabric (e.g., with one-half inch of overlapping fabric). The pad 2003a is supported by a tapered left panel 2007a of the bra 2001, which extends from a left strap 2009a down to a bottom band 2011, which is disposed below the pad 2003a and surrounds the torso of the user. The left strap 2009a is configured to extend over the left shoulder of the user and connects to a back panel 2013. The left strap 2009a can optionally include an adjuster (not shown), such as a slider, to enable the length of the left strap 2009a to be adjusted by the user. The bottom band 2011 can be formed from an elastic fabric material. The bottom band 2011 can optionally include fasteners (not shown), such as hook and eye fasteners or hook and loop or Velcro fasteners, to enable opening and closing the bottom band 2011 for putting on and removing the bra 2001 during use.

The bra 2001 also includes a pad 2003b that is contoured and configured to surround and engage the right breast of a user during use. The pad 2003b may be made from fabric, polymer, or any flexible material. The pad 2003b defines an opening or slit 2005b corresponding to the nipple area of the right breast. The opening or slit 2005b can be configured to receive therethrough a portion of a breast shield (not shown). The opening or slit 2005b can be defined by overlapping fabric (e.g., with one-half inch of overlapping fabric). The pad 2003b is supported by a tapered right panel 2007b of the bra 2001, which extends from a right strap 2009b down to the bottom band 2011. The right strap 2009b is configured to extend over the right shoulder of the user and connects to the back panel 2013. The right strap 2009b can optionally include an adjuster (not shown), such as a slider, to enable the length of the right strap 2009b to be adjusted by the user.

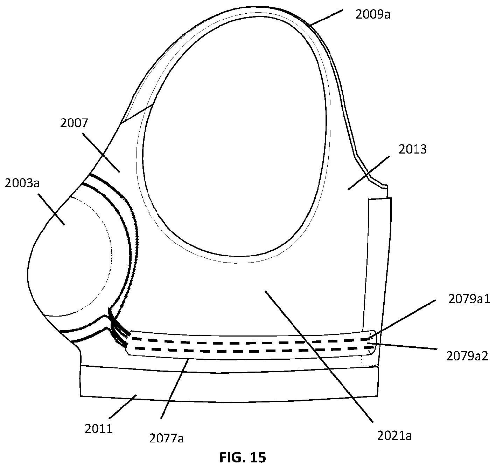

As best shown in FIG. 15, bra 2001 also includes a left-wing panel 2021a that extends from the left side of panel 2007a rearwards to the back panel 2013. The left-wing panel 2021a is configured to extend about the left side of the torso of the user and under the left arm of the user during use. The bra 2001 also includes a right-wing panel 2021b that extends from the right side of panel 2007b to the back panel 2013 similar to the left-wing panel 2021a. The right-wing panel 2021b is configured to extend about the right side of the torso of the user and under the right arm of the user during use.

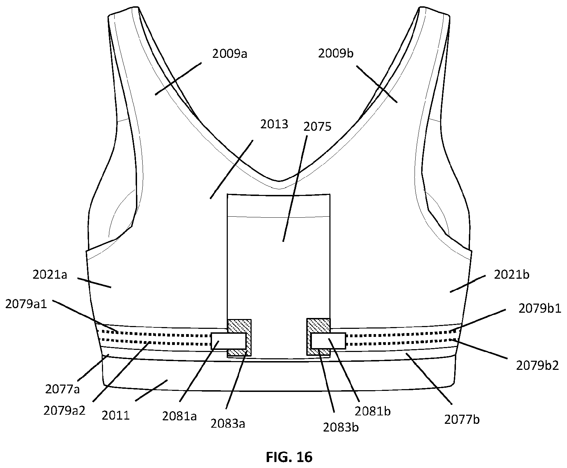

As best shown in FIG. 16, the back panel 2013 extends upward from the bottom band 2011 to the left and right straps 2009a, 2009b. The back panel 2013 also extends laterally to the left-wing and right-wing panels 2021a, 2021b.

The panels 2007a, 2007b, 2021a, 2021b, 2013 and the left and right straps 2009a, 2009b and the bottom band 2011 can be formed from fabric, polymer, or any flexible material. In embodiments, the panels 2007a, 2007b, 2021a, 2021b, 2013 and the left and right straps 2009a, 2009b and the bottom band 2011 can be formed by a three-dimensional knitting machine programmed via a digital design of the bra and supplied with desired yarn and/or other suitable material. In embodiments, the whole bra can be made of the same basic material, such as jacquard knit, with possible variations in the amount of latex or other material used in certain areas or parts of the bra for desired properties (such as elasticity or comfort).

As best shown in FIG. 14, the pad 2003a supports a plurality of cable loops configured to slide, translate, or otherwise displace relative to the pad 2003a along circumferential paths guided by an arrangement of channels that are formed integral to the pad 2003a. In FIG. 14, two channels are shown as solid lines and labeled 2017a1 and 2017a2 with respective cable loops 2019a1, 1019a2 extending therethrough. In embodiments, the channels 2017a1, 2017a2can be formed from fabric material knitted, sewn or otherwise joined to the pad 2003a, such as a tubular jacquard knit. For purposes herein, the term "cable loop" is to be understood broadly to include wire, string, monofilaments, multifilaments, twisted filaments, etc., which may be comprised of any of several materials such as metal, polymers which are substantially inelastic in tension, or other suitable materials, or combinations thereof. The circumferential paths formed by the channels 2017a1, 2017a2 (and the corresponding cable loops extending therethrough) are offset from one another in a longitudinal direction A (see FIG. 6). During use, the longitudinal direction A is intended to extend generally parallel to the sagittal plane of the user from the torso/base of the left breast to the nipple of the left breast. In this manner, the circumferential paths formed by the channels 2017a1, 2017a2 (and the corresponding cable loops extending therethrough) are spaced from one another at different positions relative to the base of the left breast or torso of the user.

The pad 2003b supports a plurality of cable loops configured to slide, translate, or otherwise displace relative to the pad 2003b along circumferential paths guided by an arrangement of channels that are formed integral to the pad 2003b. In FIG. 14, two channels are shown as solid lines and labeled 2017b1 and 2017b2 with cables loops 2019b1, 2019b2 extending therethrough. In embodiments, the channels 2017b1, 2017b2 can be formed from fabric material knitted, sewn or otherwise joined to the pad 2003b, such as a tubular jacquard knit. The circumferential paths formed by the channels 2017b1, 2017b2 (and the corresponding cable loops extending therethrough) are offset from one another in a longitudinal direction similar to direction shown in FIG. 6. During use, the longitudinal direction is intended to extend generally parallel to the sagittal plane of the user from the torso/base of the right breast to the nipple of the right breast. In this manner, the circumferential paths formed by the channels 2017b1, 2017b2 (and the corresponding cable loops extending therethrough) are spaced from one another at different positions relative to the base of the right breast or torso of the user.

### MASSAGE UNIT

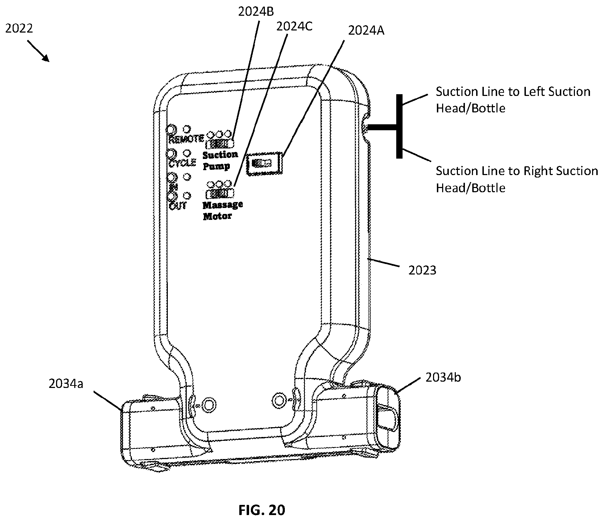

The cable loops 2019a1, 2019a2 that extend through the channels 2017a1, 2017a2 of pad 2003a as well as the cable loops 2019b1, 2019b2 that extend through the channels 2017b1, 2017b2 of pad 1003b are mechanically coupled to a massage unit 2022 as best shown in FIGS. 20 to 29. The massage unit 2022 can be configured to actuate displacement of the cable loops 2019a1, 2019a2 relative to the pad 2003a to compress the left breast and simultaneously actuate displacement of the cable loops 2019b1, 2019b2 relative to the pad 2003b to compress the right breast. The massage unit 2022 can also be configured to reverse the displacement of the cable loops 2019a1, 2019a2 relative to the pad 2003a to permit the left breast to expand and decompress and simultaneously reverse the displacement of the cable loops 2019b1, 2019b2 relative to the pad 2003b to permit the right breast to expand and decompress.

As shown in FIGS. 20, 21, 22A and 22B, the massage unit 2022 includes a housing 2023 that encloses a PCB with a controller and supporting electronics 2025, electric motor 2027 (e.g., stepper motor), at least one battery (e.g., lithium-ion battery) 2029, transmission 2031, a left rotary pull mechanism 2033a (winding spool) for the cable loops 2019a1, 2019a2 of the left pad 2003a, and a right rotary pull mechanism 2033b (winding spool) for the cable loops 2019b1, 2019b2 of the right pad 2003b. The operations of the left and right rotary pull mechanisms 2033a, 2033b are driven by the rotary output of the electric motor 2027 via the transmission 2031.

In embodiments, the front side of housing 2023 (which faces away from the user's body during use) can include a user-activated ON/OFF switch 2024A for selectively powering the unit 2022 ON and OFF as well as user-activated switches 2024B, 2024C or controlling operating parameters for the suction pump and massage motor, respectively, of the unit as shown in FIG. 20. The front side of housing 2023 can also include LED lights that display operating parameters for the unit as shown in FIG. 20.

In embodiments, the rear side of housing 2023 (which faces toward the user's body during use) can include a user-removable panel or door 2028 that provides user access to a battery compartment for the one or more batteries 2029. The user can remove the panel or door 2028 to replace the one or more batteries 2029, and then close the panel or door 2028 to enclose the one or more batteries 2020 in the battery compartment.

In embodiments, the electric motor 2027, transmission 2031 and left rotary pull mechanism 2033a can cooperate to apply one or more compression-expansion cycles to the cable loops 2019a1, 2019a2 for the left pad 2003a. Simultaneously, the electric motor 2027, transmission 2031 and right rotary pull mechanism 2033b can cooperate to apply one or more compression-expansion cycles to the cable loops 2019b1, 2019b2 for the right pad 2003b. Each compression-expansion cycle selectively displaces the cable loops to decrease the effective lengths of the cable loops that surround the breast to compress the breast, and then displaces the cable loops to increase the effective lengths of the cable loops that surround the breast to permit the breast to expand and decompress. In embodiments, each compression-expansion cycle includes compression operations that sequentially displace the cable loops to decrease the effective lengths of the cable loops that surround the breast from the inner cable loop (closer to the torso) to the outer cable (furthest from the torso) without removal of tension from the cable loops in order to apply sequential compression to the breast. Each compression-expansion cycle further includes expansion operations that displace the cable loops to increase the effective lengths of the cable loops that surround the breast to permit expansion of the breast. The sequential compression operations followed by the expansion operations as part of the compression-expansion cycle mimics massaging of the breast and can effectively move milk toward the nipple of the breast for expression therefrom.

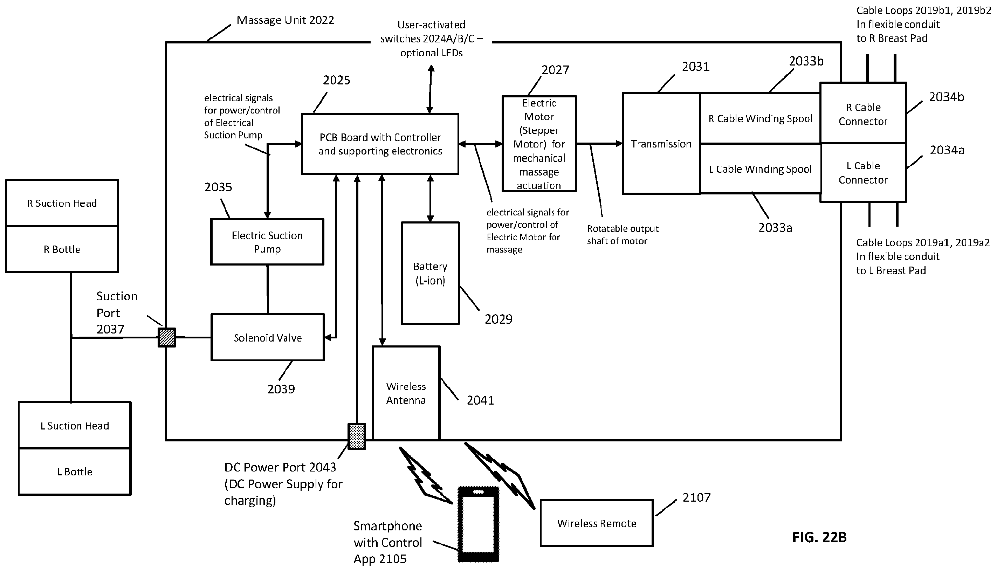

As best shown in FIG. 22B, the controller and supporting electronics of the PCB 2025 can be configured to supply electrical signals to the electric motor 2027 to provide power and control of the electric motor 2027 in driving the mechanical massage actuation of the left and right breasts as provided by cooperation of the motor 2027, transmission 2031 and the left and right rotary pull mechanisms 2033a, 2033b. The controller and supporting electronics of the PCB 2025 can also be configured to supply electrical signals to an electric suction pump 2035 that is enclosed by the housing 2023 to control the operation of the suction pump 2035 in supplying suction to external suction head and bottle assemblies for the left and right breasts via a suction port 2037 in the housing 2023. A T-shaped connector and suction lines can be fluidly coupled to this suction port 2027 to connect to the external suction head and bottle assembles for the left and right breasts. In this configuration, the negative pressure (suction) generated by the suction pump 2035 of the massage unit 2022 can be supplied to the external suction head and bottle assemblies for the left and right breasts. The controller and supporting electronics of the PCB 2025 can also be configured to supply electrical signals to a solenoid valve 2039 that is enclosed by housing 2023. The controller and supporting electronics of the PCB 2025 can configure the solenoid valve 2039 to vent the suction port/lines to ambient during the operation of the unit 2022.

In embodiments, the controller and supporting electronics of the PCB 2025 can be configured to receive electrical signals from the user-activated switches 2024A, 2024B, 2024C as described above to control the operation of the unit. The controller and supporting electronics of the PCB 2025 can also be configured to supply electrical signals to optional LEDS as described above to indicate operational status of the unit.

In embodiments, the housing 2023 can also enclose a wireless antenna 2041 that interfaces to the controller and supporting electronics of the PCB 2025. The controller and supporting electronics of the PCB 2025 can cooperate with the wireless antenna 2041 to provide a wireless communication link to a computing device. The wireless communication link can support a standardized wireless communication protocol such as any one of a number of Bluetooth^{®} protocols (e.g., Bluetooth^{®} v1.0 to v1.08, Bluetooth^{®} v1.1, Bluetooth^{®} v1.2, and Bluetooth^{®} v2.0), or any one of a number of Wi-Fi or IEEE 802.11 protocols, or other wireless data communication protocols. The computing device can be a smartphone, smartwatch, tablet computer, or another computing device. For example, FIG. 22B depicts a smartphone labeled 2105. Alternatively or additionally, the computing device can be a wireless remote device, which is labeled 2107 in FIG. 22B. The computing device may execute software (e.g., an application, a.k.a., an "app") that graphically displays an operational interface to a user. The operational interface may be used to configure and/or control the operating parameters of the massage unit 2022, including operating parameters of the electric motor 2027 that drives the massage actuation of the massage unit 2022 and operating parameters of the suction pump 2035 of the massage unit 2022. The operating parameters may be saved locally on the computing device or remotely (for example, in cloud data storage) during use for retrieval and/or analysis.

Furthermore, housing 2023 can include a port 2043 for receiving DC electrical power that charges the one or more batteries 2029. When charged, the one or more batteries 2029 can be used for supply of electrical power to the electrical components of the unit 2022, including the controller and supporting electronics of the PCB 2025, the electric motor 2027, the suction pump 2035 and the solenoid valve 2039. The electrical power can be regulated for supply to the electrical components of the unit as needed.

Referring back to FIGS. 14 to 16, the back panel 2013 can include a pocket or sleeve 2075 formed from fabric and similar to the pocket or sleeve 24a as shown in FIGS. 3 and 6. In embodiments, the fabric of the pocket or sleeve 2075 can be a jacquard knit that is knitted or sewn or otherwise joined to the back panel 2013. The dimensions and material of the pocket or sleeve 2075 can be configured to enclose and secure at least part of the massage unit 2022 unit during use. For example, the dimensions and material of the pocket or sleeve 2075 can be configured to retain and hold the massage unit 2022 adjacent or near the bottom band 2011. In this configuration, the bra 2001 can support the massage unit 2022 in a position adjacent the upper back of the user, which enables the massage unit 2022 to be worn and used in a discrete manner. In embodiments, an opening leading into the pocket or sleeve 2075 extends laterally across the top side of the pocket or sleeve 2075. The top side opening can be sized to accommodate the lateral dimension of the massage unit 2022 to enable the massage unit (or part thereof) to be placed into the pocket or sleeve 2075 or removed from the pocket or sleeve 2075.

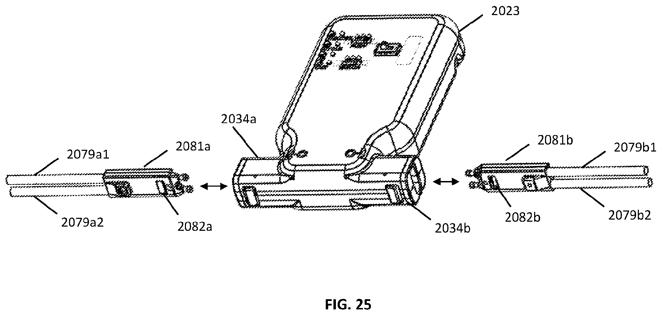

Furthermore, the left-wing panel 2021a is provided with a fabric channel 2077a that extends along the bottom portion of the left-wing panel 2021a generally parallel to the bottom band 2011 to the bottom of the pocket or sleeve 2075. The fabric channel 2077a can be formed from fabric material knitted, sewn or otherwise joined to the left-wing panel 2021a, such as a tubular jacquard knit. Similarly, the right-wing panel 2021b is provided with a fabric channel 2077b that extends along the bottom portion of the right-wing panel 2021b generally parallel to the bottom band 2011 to the bottom of the pocket or sleeve 2075. The fabric channel 2077b can be formed from fabric material knitted, sewn or otherwise joined to the right-wing panel 2021b, such as a tubular jacquard knit. The channel 2077a supports conduit/tubing 2079a1, 2079a2 (shown as dotted lines) that house the cable loops 2019a1, 2019a2 for the left breast pad 2003a. The channel 2077b supports conduit/tubing 2079b1, 2079b2 (shown as dotted lines) that house the cable loops 2019b1, 2019b2 for the right breast pad 2003b. The conduit/tubing 2079a1, 2079a2, 2079b1, 7079b2 can be formed from flexible material that generally conforms to the curvature of the torso of the user during use. In embodiments, the conduit/tubing 2079a1, 2079a2, 2079b1, 7079b2 can have multiple internal passageways/lumens that receive and accommodate the corresponding cable loops and permit for relative movement of one or more cable loops therein during the massage actuation as described herein. The conduit/tubing 2079a1, 2079a2 can terminate at a male connector 2081a that is adapted to mate/lock to a corresponding female connector 2034a of the massage unit 2022 as best shown in FIGS. 25-29. The conduit/tubing 2079b1, 2079b2 can terminate at a male connector 2081b that is adapted to mate/lock to a corresponding female connector 2034b of the massage unit 2022 as best shown in FIGS. 25-29. The pocket or sleeve 2075 can be configured with optional cutouts 2083a, 2083b that provide user access to the terminal connectors 2081a, 2081b. Cutout 2083a enables the user to mate connector 2081a to connector 2034a of the massage unit 2022 when the massage unit 2022 is placed inside the pocket or sleeve 2075. Cutout 2083b enables the user to mate connector 2081b to connector 2034b of the massage unit 2022 when the massage unit 2022 is placed inside the pocket or sleeve 2075.

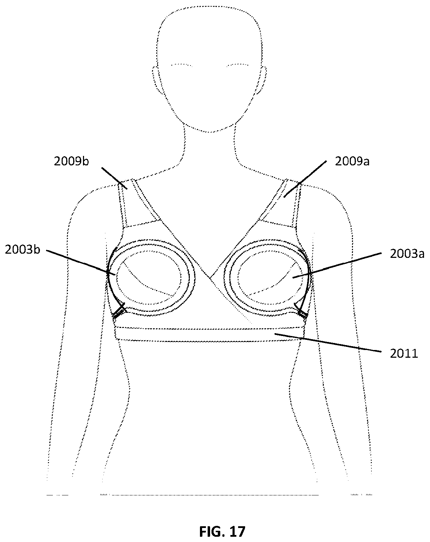

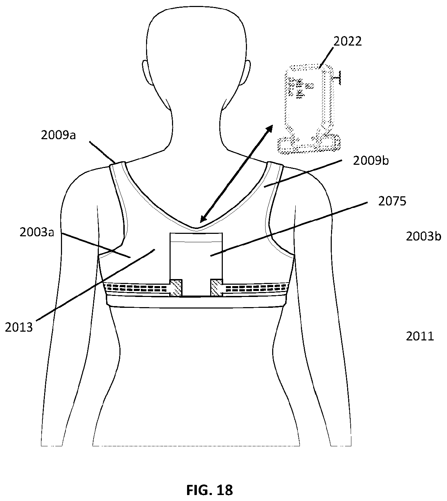

FIGS. 17 to 19 illustrate a user wearing the bra 2001. FIG. 18 illustrates insertion of the massage unit 2022 into the pocket or sleeve 2075 (or removal therefrom). Such insertion/removal can be performed by the user when the user is wearing the bra 2001. Additionally or alternatively, such insertion/removal can be performed by the user when the user is not wearing the bra 2001.

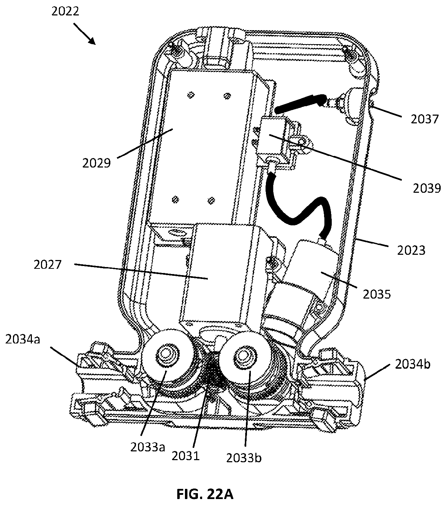

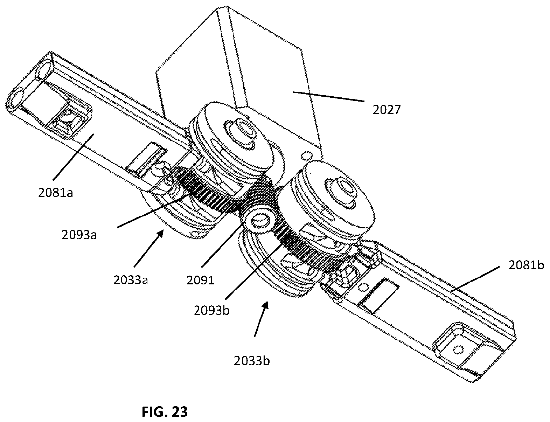

In the embodiment of FIGS. 14 to 29, the massage unit 2022 includes a transmission 2031 driven by the output shaft of the electric motor 2027. In embodiments, the transmission can be embodied by a worm gear 2091 mounted to the output shaft of the motor 2027. The worm gear 2091 interfaces to drive gears 2093a, 2093b that are coaxially mounted to the left and right rotational pull mechanism (winding spools) 2033a, 2033b. The worm gear 2091 is disposed between the left and right rotational pull mechanism (winding spools) 2033a, 2033b with the rotational axis of the worm gear orthogonal to the rotational axes of the left and right rotational pull mechanism (winding spools) 2033a, 2033b as shown in FIGS. 22A and 23. The rotation of the worm gear 2091 drives rotation of the drive gears 2093a, 2093b together with the corresponding left and right rotational pull mechanism (winding spools) 2033a, 2033b.

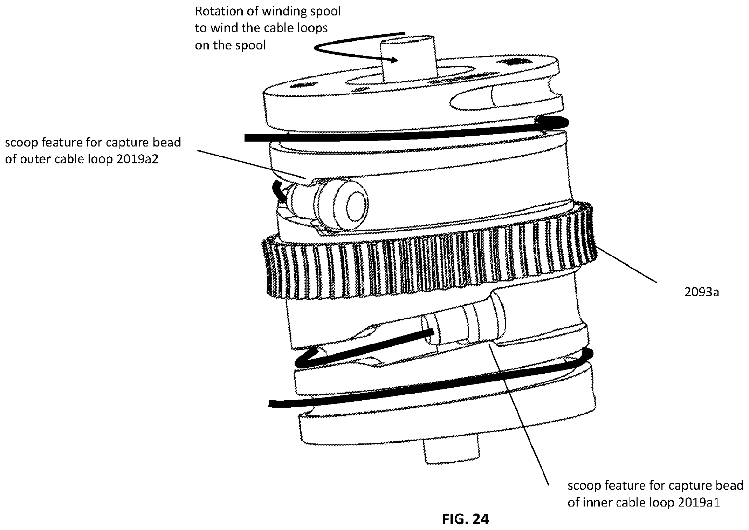

The left rotational pull mechanism 2033a is a two-part winding spool, which has one part for the inner cable loop 2019a1 and another part for the outer cable loop 2019a2, where the winding spool parts are disposed on opposite sides of the drive gear 2093a. The cable loops 2019a1, 2019a2 are terminated by corresponding capture beads located outside the body of the male connector 2081a (FIG. 24). Similar to the embodiment of the rotary pull mechanism of FIGS. 11A to 11D as described above, the winding spool parts include scoop features that capture and engage the capture beads of the inner and outer cable loops 2019a1, 2019a2 during certain rotation of the winding spool. When a capture bead is engaged by the corresponding scoop feature, further rotation of the winding spool causes the corresponding cable loop to wind around the winding spool part and decrease the effective length of the cable loop. In order to accommodate sequential pulling of the outer cable loop 2019a2 after the inner cable loop 2019a1, the scoop feature for the inner cable loop 2019a1 can be offset from the scoop feature for the outer cable loop 2109a2 by a predefined lead angle of rotation of the winder spool. This feature is shown in detail in FIG. 24.

Similarly, the right rotational pull mechanism 2033b is a two-part winding spool, which has one part for the inner cable loop 2019b1 and another part for the outer cable loop 2019b2, where the winding spool parts are disposed on opposite sides of the drive gear 2093b. The cable loops 2019b1, 2019b2 are terminated by corresponding capture beads located outside the body of the male connector 2081b. The winding spool parts include scoop features that capture and engage the capture beads of the inner and outer cable loops 2019b1, 2019b2 during certain rotation of the winding spool. When a capture bead is engaged by the corresponding scoop feature, further rotation of the winding spool causes the corresponding cable loop to wind around the winding spool part and decrease the effective length of the cable loop. In order to accommodate sequential pulling of the outer cable loop 2019b2 after the inner cable loop 2019b1, the scoop feature for the inner cable loop 2019b1 can be offset from the scoop feature for the outer cable loop 2109b2 by a predefined lead angle of rotation of the winder spool.

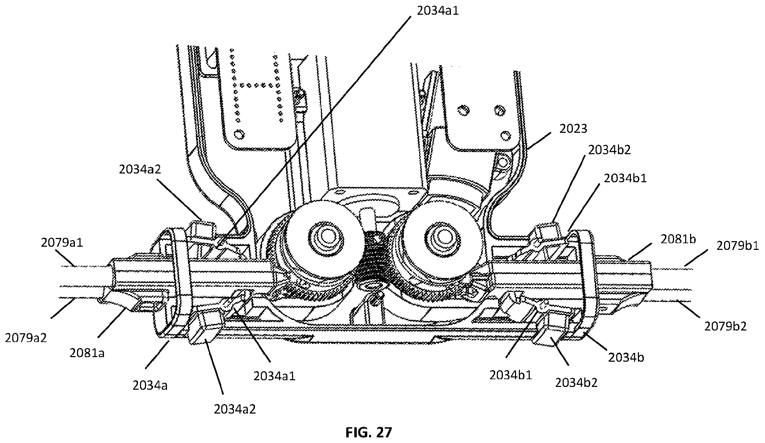

When the male connector 2018a is connected (or reconnected) to the female connector 2034a of the massage unit 2022 (FIG. 27), the capture beads for the inner and outer cable loops 2019a1, 2019a2 for the left breast pad 2003a can be positioned in a neutral position adjacent the respective winding spool parts of the left rotational pull mechanism 2033a. In this neutral position, no tension (or little tension) is applied to any of the cable loops and the left breast is not being compressed. When the winding spool parts of left rotational pull mechanism 2033a rotate under actuation provided by the electric motor 2027 and gear transmission 2031, such movement causes the scoop features of the winding spool parts to selectively capture and wind the corresponding cable loops 2019a1, 2019a2 about the winding spool parts to decrease the effective length of the corresponding cable loops that surrounds the left breast, which results in radial inward displacement of pad 2003a and compression of the left breast of the user. Owing to the arrangement of the angular offset between the scoop features, the timing and sequence of the rotational capture and winding of the corresponding cable loops 2019a1, 2019a2 and the resulting decrease in the effective lengths of the corresponding cable loops and application of tension to the cable loops and breast compression can be controlled during each rotational stroke of the left rotational pull mechanism 2033a. This stroke corresponds to the compression phase of the compression-expansion cycle for the left breast. Thereafter, the massage unit 2022 may be configured to begin an expansion stroke of the winding spool parts of the left rotational pull mechanism 2033a and an expansion phase of the compression-expansion cycle, which increase the effective lengths of both the inner and outer cable loops 2019a1, 2019a2 that surround the left breast to remove tension in such cable loops, by rotating the scoop features of the winding spool parts in the opposite rotational sense that returns the capture beads of the cable loops 2019a1, 2019a2 back to their neutral positions, completing the compression-expansion cycle for the left breast.

Similarly, when the male connector 2018b is connected (or reconnected) to the female connector 2034b of the massage unit 2022 (FIG. 27), the capture beads for the inner and outer cable loops 2019b1, 2019b2 for the right breast pad 2003b can be positioned in a neutral position adjacent the respective winding spool parts of the left rotational pull mechanism 2033b. In this neutral position, no tension (or little tension) is applied to any of the cable loops and the right breast is not being compressed. When the winding spool parts of right rotational pull mechanism 2033b rotate under actuation provided by the electric motor 2027 and gear transmission 2031, such movement causes the scoop features of the winding spool parts to selectively capture and wind the corresponding cable loops 2019b1, 2019b2 about the winding spool parts to decrease the effective length of the corresponding cable loops that surrounds the right breast, which results in radial inward displacement of pad 2003b and compression of the right breast of the user. Owing to the arrangement of the angular offset between the scoop features, the timing and sequence of the rotational capture and winding of the corresponding cable loops 2019b1, 2019b2 and the resulting decrease in the effective lengths of the corresponding cable loops and application of tension to the cable loops and breast compression can be controlled during each rotational stroke of the right rotational pull mechanism 2033b. This stroke corresponds to the compression phase of the compression-expansion cycle for the right breast. Thereafter, the massage unit 2022 may be configured to begin an expansion stroke of the winding spool parts of the right rotational pull mechanism 2033b and an expansion phase of the compression-expansion cycle, which increase the effective lengths of both the inner and outer cable loops 2019b1, 2019b2 that surround the right breast to remove tension in such cable loops, by rotating the scoop features of the winding spool parts in the opposite rotational sense that returns the capture beads of the cable loops 2019b1, 2019b2 back to their neutral positions, completing the compression-expansion cycle for the right breast.

The gear transmission 2031 drives the left and right rotational pull mechanisms 2033a 2033b in a synchronous manner such that the compression-expansion cycle for the left breast occurs simultaneously with the compression-expansion cycle for the left breast.

Such compression-expansion cycles can be repeated multiple times to facilitate breast milk expression. Thus, based on the compression sequence, the inner cable loops will compress the breasts first, followed by compression of the breasts by the outer cable loops, such that the breasts will be compressed progressively in an outward direction (e.g., longitudinal direction A in FIG. 6) from the base of the breast toward the nipple area of the breast. Also, it will be appreciated that during each compression stroke, the inner cable loops will be in tension for a longer period of time than the outer cable loops.

In other embodiments, the configuration of the scoop features of the winding spool parts and/or the rotational speed of the electric motor 2027 can be readily adapted to provide variable delays in the peak of the compression cycle or variable speeds of compression/decompression cycles.

In alternate embodiments, the capture beads and corresponding scoop features of the rotary winding spool parts can be replaced with other mechanical structures that detachably couple the cable loops to the winding spool with delayed winding of the outer cable loop relative to the inner cable loop for sequential tensioning of the inner and outer cable loops as described herein.

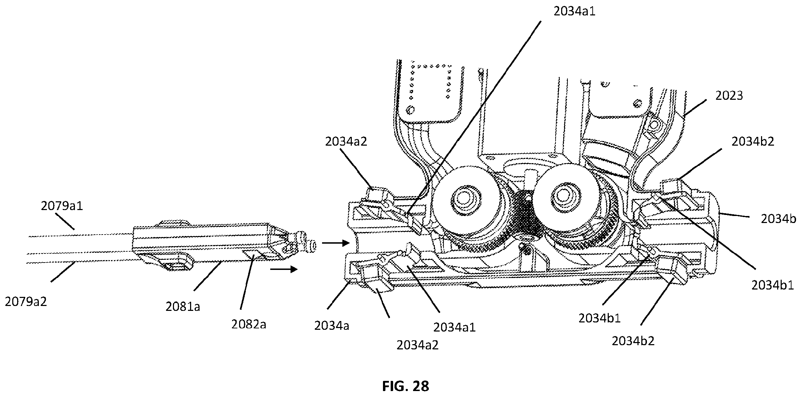

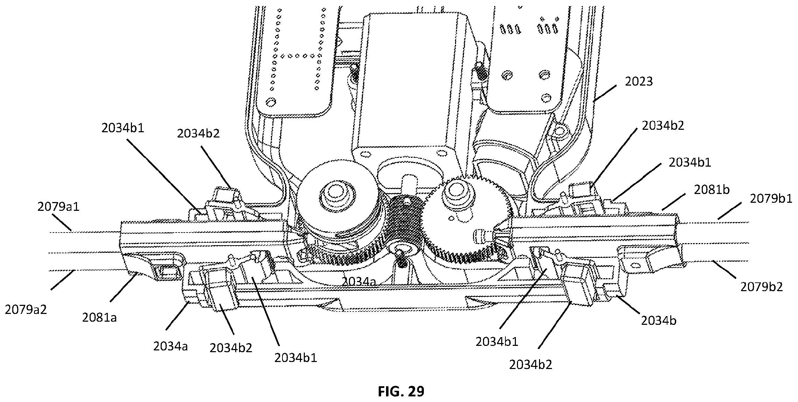

As best shown in FIGS. 25-29, the conduit/tubing 2079a1, 2079a2 can terminate at a male connector 2081a that is adapted to mate/lock to a corresponding female connector 2034a of the massage unit 2022. In embodiments, the male connector 2081a can include opposed recesses 2082a in top and bottom surfaces (one shown in FIGS. 25 and 28) that receive and mate to corresponding spring-clips 2034a1 of the female connector 2034a as best shown in FIGS 27 to 29. The spring-clips 2034a1 have exposed knob-like elements 2034a2 that can be pressed by a user to disengage the spring-clips 2034a1 from the recesses 2082a of the male connector 2081a to disconnect and/or remove the male connector 2081a from the female connector 2034a. Similarly, the male connector 2081b can include opposed recesses 2082b in top and bottom that receive and mate to corresponding spring-clips 2034b1 of the female connector 2034b as best shown in FIGS 27 to 29. The spring-clips 2034b1 have exposed knob-like elements 2034b2 that can be pressed by a user to disengage the spring-clips 2034b1 from the recesses 2082b of the male connector 2081b to disconnect and/or remove the male connector 2081b from the female connector 2034b. FIG. 25 shows the male connector 2081a disconnected from the female connector 2034a and the male connector 2081b disconnected from the female connector 2034b. FIGS. 27 and 29 shows the male connector 2081a mated and locked to the female connector 2034a and the male connector 2081b mated and locked to the female connector 2034b.

In other embodiments, one or more mechanical structures, components and features of the massage unit and bra of FIGS. 14 to 29 can be incorporated into the embodiments of FIGS. 1 to 14 as described herein.

In the embodiments described herein, the cable loops can be routed through beads, tubular structure, or other structural elements that are part of the channels of the respective pad of the bra. Each cable loop is configured to slide freely through openings defined in the corresponding beads or through the tubular structure or other structural elements. The beads may have a flat outer surface, or they may have other outer shapes, such as cylindrical and round (e.g., spherical). The beads may be secured to the pad with adhesive (e.g., glue) or by sewing. Also, the aforementioned tubular channels may be a single continuous channel, or a broken tubular channel circumferentially spaced about the pad.

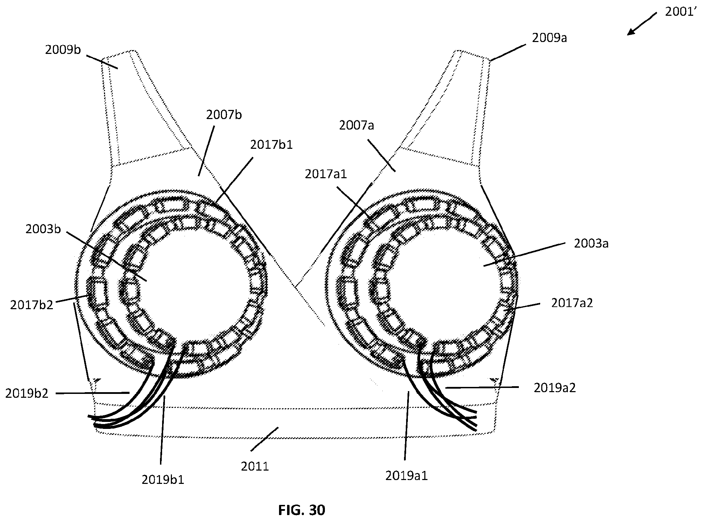

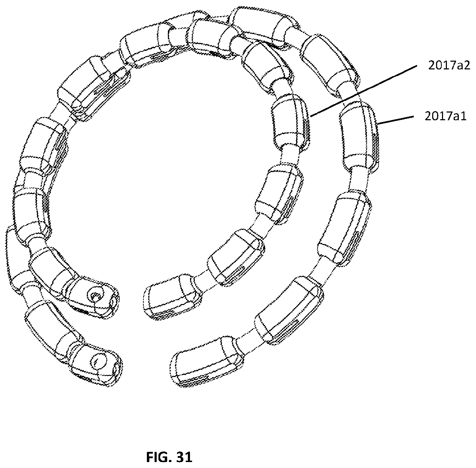

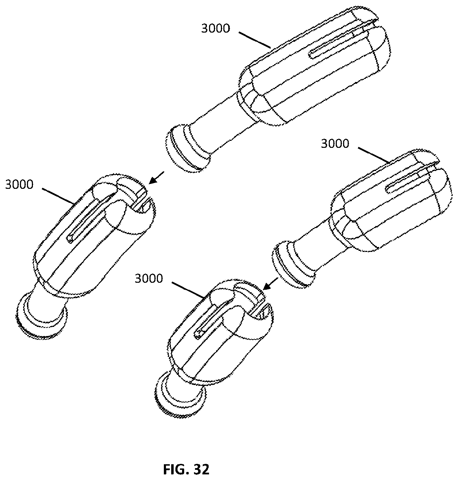

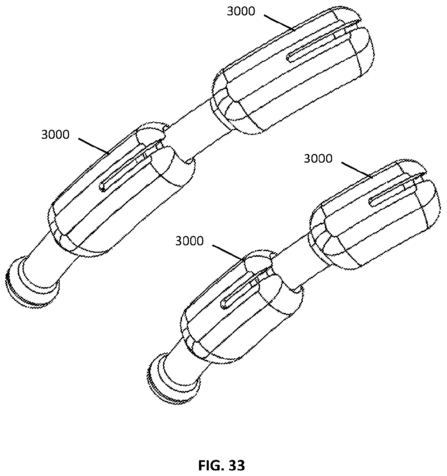

In the embodiments described herein, the pads of the bra can employ continuous channels for guiding cable loops where the continuous channels are formed from interlocking bead-like structures. In one embodiment illustrated in FIGS. 30 to 33, channels 2017a1, 2017a2 for the cable loops 2019a1, 2019a2 of the left breast pad 2003a are formed from interlocking bead-like structures 3000, and channels 2017b1, 2017b2 for the cable loops 2019b1, 2019b2 of the right breast pad 2003b are formed from the same interlocking bead-like structures 3000. The bead-like structures 3000 are each formed for a generally tubular body with a shallow arc-like profile along the length of the tubular body. The tubular body includes a larger-diameter tail section (with opposed longitudinal slits) that extends to a smaller-diameter neck portion that terminates at a disc-like head portion as best shown in FIG. 32. The disk-like head portion can be inserted into and secured with the larger diameter tail section of the adjacent bead-like structure as shown in FIG. 33 to construct the continuous channels from interlocking bead-like structures. The disk-like head portion is somewhat spherical in shape and this allows for omnidirectional angular displacement of one bead-like structure relative to the next bead-like structure such that as the diameter of the loop changes, the bead-like structures can pivot about one another to accommodate the diameter change. Additionally, this same aspect of the interlocking bead-like structures allows for out-of-plane conforming of the interlocking bead-like structures to the geometry of the user's breasts for overall better comfort. The longitudinal slits of the larger-diameter tail section can be configured to permit the larger-diameter tail section to elastically deform to allow the disk-like head portion of the adjacent bead-like structure to be inserted into (e., snapped into place within) the larger diameter tail section. The larger diameter section can then return back to its original shape (by the elastic deformation) such that the larger-diameter tail section secures and holds the head portion of the adjacent bead-like structure in the larger-diameter tail section as best shown in FIG. 33.

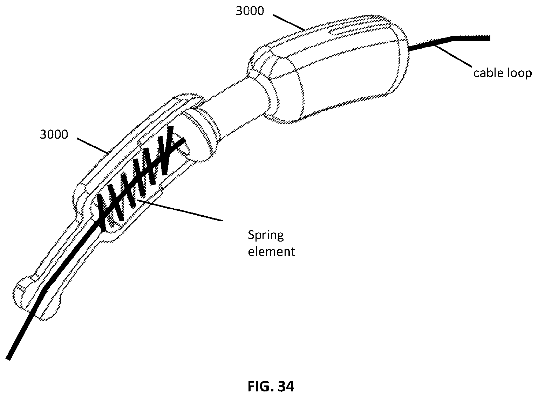

In embodiments, the larger-diameter tail section of each interlocking bead-like structure 3000 can enclose a spring element (such as a helical coil spring) as shown in FIG. 34. The disk-like head portion can be retained within the tail section against the expansion force of the spring element by a smaller diameter opening to the tail section (smaller than the majority of the bore of the tail section) that surrounds the disk-like head portion when the bead chain is at full extension (neutral position). In this embodiment, the spring element provides a resilient bias force that pushes against the disk-like head portion of the adjacent bead-like structure. The resilient bias force can aid in increasing the effective length of the cable loop guided by the interlocking bead-like structure in the expansion phase of the cyclic compression-expansion cycles of massage and remove tension in the cable loop and permit expansion/decompression of the breast. The resilient bias force can also aid in returning the capture beads that terminate the corresponding cable loop back to its neutral position to complete the compression-expansion cycle as described herein.

In the embodiments described herein, the massage unit(s) of the system can employ more than two cable loops that surround a breast, such as three or more cable loops, and apply compression-expansion cycles to the three or more cable loops in a manner similar to that described above.

In the embodiments described herein, the number and longitudinal spacing of the cable loops may be determined based on the size of the breast and the timing of the compression-expansion cycles as described herein. Also, the number and circumferential spacing of the cable loops may be determined based on the size of the breast.

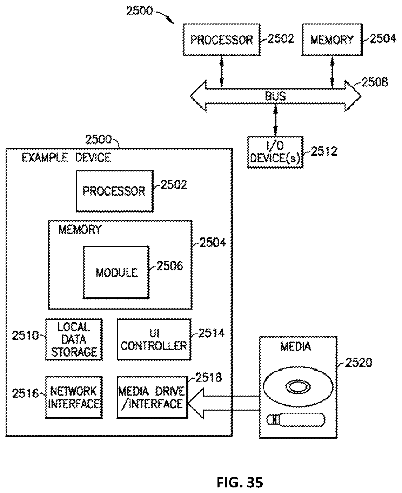

Figure 35 illustrates an example device 2500, with a processor 2502 and memory 2504 that can be configured to implement various embodiments of a controller or computing device as described herein. Memory 2504 can include one or more forms of volatile data storage media such as random-access memory (RAM), and/or one or more forms of nonvolatile storage media (such as read-only memory (ROM), flash memory, and so forth).

Device 2500 is one example of a computing device or programmable device and is not intended to suggest any limitation as to scope of use or functionality of device 2500 and/or its possible architectures. For example, device 2500 can comprise one or more computing devices, programmable logic controllers (PLCs), etc.

Further, device 2500 should not be interpreted as having any dependency relating to one or a combination of components illustrated in device 2500. For example, device 2500 may be a mobile device such as a smartphone, smartwatch, notepad, laptop, desktop computer, etc., or any combination or accumulation thereof.

Device 2500 can also include a bus 2508 configured to allow various components and devices, such as processors 2502, memory 2504, and local data storage 2510, among other components, to communicate with each other.

Bus 2508 can include one or more of any of several types of bus structures, including a memory bus or memory controller, a peripheral bus, an accelerated graphics port, and a processor or local bus using any of a variety of bus architectures. Bus 2508 can also include wired and/or wireless buses.

Local data storage 2510 can include fixed media (e.g., RAM, ROM, a fixed hard drive, etc.) as well as removable media (e.g., a flash memory drive, a removable hard drive, optical disks, magnetic disks, and so forth).

One or more input/output (I/O) device(s) 2512 may also communicate via a user interface (UI) controller 2514, which may connect with I/O device(s) 2512 either directly or through bus 2508.

In one possible implementation, a network interface 2516 may communicate outside of device 2500 via a connected network.

A media drive/interface 2518 can accept removable tangible media 2520, such as flash drives, optical disks, removable hard drives, software products, etc. In one possible implementation, logic, computing instructions, and/or software programs comprising elements of module 2506 may reside on removable media 2520 readable by media drive/interface 2518.

Various processes of the present disclosure or parts thereof can be implemented by instructions and/or software programs that are elements of module 2506. Such instructions and/or software programs may reside on removable media 2520 readable by media drive/interface 2518 as is well known in the computing arts.

In one possible embodiment, input/output device(s) 2512 can allow a user (such as a human annotator) to enter commands and information to device 2500, and also allow information to be presented to the user and/or other components or devices. Examples of input device(s) 2512 include, for example, a touch screen, a keyboard, a cursor control device (e.g., a mouse), a microphone, a scanner, and any other input devices known in the art. Examples of output devices include a display device (e.g., an LCD or other display), speakers, and so on. The touch screen and display device can be part of an integrated touch screen display device as is well known.

Various processes or parts of the workflow of the present disclosure may be described herein in the general context of software or program modules, or the techniques and modules may be implemented in pure computing hardware. Software generally includes routines, programs, objects, components, data structures, and so forth that perform particular tasks or implement particular abstract data types. An implementation of these modules and techniques may be stored on or transmitted across some form of tangible computer-readable media. Computer-readable media can be any available data storage medium or media that is tangible and can be accessed by a computing device. Computer readable media may thus comprise computer storage media. "Computer storage media" designates tangible media, and includes volatile and non-volatile, removable, and non-removable tangible media implemented for storage of information such as computer readable instructions, data structures, program modules, or other data. Computer storage media include, but are not limited to, RAM, ROM, EEPROM, flash memory or other memory technology, CD-ROM, digital versatile disks (DVD) or other optical storage, magnetic cassettes, magnetic tape, magnetic disk storage or other magnetic storage devices, or any other tangible medium which can be used to store the desired information, and which can be accessed by a computer.

In embodiments, any one or any portion or all of the steps or operations of the workflow as described above can be performed by a processor. The term "processor" should not be construed to limit the embodiments disclosed herein to any particular device type or system. For example, the processor may include a microprocessor, microcontroller, digital signal processor, or general purpose computer for executing any of the methods and processes described above.

The controller or control device may further include a memory such as a semiconductor memory device (e.g., a RAM, ROM, PROM, EEPROM, or Flash-Programmable RAM), a magnetic memory device (e.g., a diskette or fixed disk), an optical memory device (e.g., a CD-ROM), a PC card (e.g., PCMCIA card), or other memory device.

Some of the methods and processes described above, can be implemented as computer program logic for use with the computer processor. The computer program logic may be embodied in various forms, including a source code form or a computer executable form. Source code may include a series of computer program instructions in a variety of programming languages (e.g., object code, an assembly language, or a high-level language such as C, C++, or JAVA). Such computer instructions can be stored in a non-transitory computer readable medium (e.g., memory) and executed by the computer processor. The computer instructions may be distributed in any form as a removable storage medium with accompanying printed or electronic documentation (e.g., shrink wrapped software), preloaded with a computer system (e.g., on system ROM or fixed disk), or distributed from a server or electronic bulletin board over a communication system (e.g., the Internet or World Wide Web).

Alternatively or additionally, the processor may include discrete electronic components coupled to a printed circuit board, integrated circuitry (e.g., Application Specific Integrated Circuits (ASIC)), and/or programmable logic devices (e.g., a Field Programmable Gate Arrays (FPGA)). Any of the methods and processes described above can be implemented using such logic devices.

While the invention has been described with respect to a limited number of embodiments, the scope of the invention is limited only by the attached claims.

## Claims

1. A breast milk expression apparatus comprising:
a bra (2001) comprising a first pad (2003a) configured to surround the left breast of a user and a second pad (2003b) configured to surround the right breast of the user;
a first plurality of cable loops (2019a1, 2019a2) with portions that extend circumferentially about an outer surface of the first pad (2003a) and that are displaceable relative to the first pad (2003a);
a second plurality of cable loops (2019b1, 2019b2) with portions that extend circumferentially about an outer surface of the second pad (2003b) and that are displaceable relative to the second pad (2003b); and
a unit (2022) configured to operably couple to one or both of the first plurality of cable loops (2019a, 2019a2) and the second plurality of cable loops (2019b1, 2019b2) and apply compression-expansion cycles to one or both of the first and second pads (2003a, 2003b) by adjusting effective length of cable loops operably coupled thereto;
**characterized in that:**
the bra (2001) has at least one integral pocket (2075) with an opening that is configured to removably receive the unit (2022) such that the pocket (2075) encloses and secures at least part of the unit (2022) during use;
the unit (2022) comprises a housing (2023), wherein the pocket (2075) is sized to enclose and secure at least part of the housing (2023);
the housing (2023) encloses at least one battery (2029), an electric motor (2027) powered by electrical power supplied by the at least one battery (2029), and at least one printed circuit board with a controller and supporting electronics (2025);
the electrical motor (2029) is configured to drive at least one massage actuator to apply the compression-expansion cycles;
the housing (2023) of the unit is further configured to enclose first and second massage actuators (2033a, 2033b) driven by the electric motor (2027), the first massage actuator (2033a) being configured to releasably couple to the first plurality of cable loops (2019a1, 2019a2) to apply compression-expansion cycles to the first pad (2003a), and the second massage actuator (2033b) being configured to releasably couple to the second plurality of cable loops (2019b1, 2019b2) to apply compression-expansion cycles to the second pad (2003b); and
the at least one pocket comprises a single pocket (2075) integral to a back panel (2013) of the bra.

2. A breast milk expression apparatus according to claim 1, wherein:
the pocket (2075) is formed from fabric that is knitted or sewn or otherwise joined to or integrated into the bra.

3. A breast milk expression apparatus according to claim 1, wherein:
the housing (2023) further encloses a vacuum pump (2035) powered by electrical power supplied by the at least one battery (2029), the vacuum pump (2035) for providing suction to an external suction head and bottle assembly for one or both of the left and right breast of the user during the compression-expansion cycles; and/or
the housing (2023) further encloses a solenoid valve (2039) configured to selectively vent to atmosphere to release suction.

4. A breast milk expression apparatus according to claim 1, wherein:
the housing (2023) includes a removable door (2028) for installing or replacing the at least one battery (2029); and/or
the supporting electronics (2025) include electrical circuitry (2043) configured to charge the at least one battery (2029) using DC power supplied by an external DC power source; and/or
the housing (2023) further encloses a wireless antenna (2041) and electronics configured to provide wireless communication between the unit (2022) and another device for controlling operation of the unit (2022).

5. A breast milk expression apparatus according to claim 1, wherein:
the first and second massage actuators each include a rotary pull mechanism (2033a, 2033b).

6. A breast milk expression apparatus according to claim 1, wherein:
the first and second massage actuators comprise rotary pull mechanisms driven (2033a, 2033b) by the electric motor (2027); and
the rotary pull mechanisms (2033a, 2033b) employ multi-part winding spools with gears (2093a, 2093b) that interface to a worm gear (2091)_that is rotatably driven by the electric motor (2027).

7. A breast milk expression apparatus according to claim 6, wherein at least part of the first plurality of cable loops (2019a1, 2019a2) and at least part of the second plurality of cable loops (2019b1, 2019b2) terminate with elements (1113a, 1113b) that are captured by corresponding scoop features (1115a, 1115b) of the multi-part winding spools.

8. A breast milk expression apparatus according to claim 7, wherein certain scoop features (1115a, 1115b) of the multi-part winding spools are offset from one another at predefined angles of rotation for sequential winding operations by the multi-part winding spools.

9. A breast milk expression apparatus according to claim 1, further comprising:
first tubing (2079a1, 2079a2) for the first plurality of cable loops (2019a1, 2019a2), the first tubing (2079a1, 2079a2) extending through or supported by fabric (2077a) disposed along a left-wing panel (2021a) of the bra; and
second tubing (2079b1, 7079b2) for the second plurality of cable loops (2019b1, 2019b2), the second tubing (2079b1, 7079b2) extending through or supported by fabric (2077b) disposed along a right-wing panel (2021b) of the bra.

10. A breast milk expression apparatus according to claim 9, wherein:
the housing (2023) further includes a first cable connector (2034a) disposed adjacent the first massage actuator (2033a) and a second cable connector (2034b) disposed adjacent the second massage actuator (2033b);
the first tubing (2079a1, 2079a2) terminates at a connector (2081a) that is configured to selectively mate and lock to the first cable connector (2034a); and
the second tubing (2079b1, 7079b2) terminates at a connector (2081b) that is configured to selectively mate and lock to the second cable connector.

11. A breast milk expression apparatus according to claim 1, wherein:
the first pad (2003a) comprises a first set of interlocking bead-like structures (3000) that define passageways (2017a1, 2017a2) for sections of the first plurality of cable loops (2019a1, 2019a2); and
the second pad (2003b) comprises a second set of interlocking bead-like structures (3000) that define passageways (2017b1, 2017b2) for sections of the second plurality of cable loops (2019b1, 2019b2).

12. A breast milk expression apparatus according to claim 11, wherein:
the interlocking bead-like structures (3000) of the first set include interior spring elements that aid in expansion of the first plurality of cable loops (2019a1, 2019a2); and
the interlocking bead-like structures (3000) of the second set include interior spring elements that aid in expansion of the second plurality of cable loops (2019b1, 2019b2).

13. A breast milk expression apparatus according to claim 11, wherein:
the interlocking bead-like structures (3000) of the first set each comprise a generally tubular body with a shallow arc-like profile along the length of the tubular body, wherein the tubular body includes a larger-diameter tail section that extends to a smaller-diameter neck portion that terminates at a disc-like head portion, and the disk-like head portion is preferably inserted into and secured with the larger diameter tail section of the adjacent bead-like structure to construct the continuous channels (2017a1, 2017a2) from interlocking bead-like structures of the first set; and
the interlocking bead-like structures (3000) of the second set each comprise a generally tubular body with a shallow arc-like profile along the length of the tubular body, wherein the tubular body includes a larger-diameter tail section that extends to a smaller-diameter neck portion that terminates at a disc-like head portion, and the disk-like head portion is preferably inserted into and secured with the larger diameter tail section of the adjacent bead-like structure to construct the continuous channels (2017b1, 2017b2) from interlocking bead-like structures of the second set.

## Patentansprüche

1. Ein Gerät zum Abpumpen von Muttermilch, das Folgendes beinhaltet:
einen Büstenhalter (2001), beinhaltend ein erstes Polster (2003a), das konfiguriert ist, um die linke Brust einer Benutzerin zu umgeben, und ein zweites Polster (2003b), das konfiguriert ist, um die rechte Brust der Benutzerin zu umgeben;
eine erste Vielzahl von Kabelschleifen (2019a1, 2019a2) mit Abschnitten, die sich in Umfangsrichtung um eine Außenfläche des ersten Polsters (2003a) erstrecken und die relativ zu dem ersten Polster (2003a) verschiebbar sind;
eine zweite Vielzahl von Kabelschleifen (2019b1, 2019b2) mit Abschnitten, die sich in Umfangsrichtung um eine Außenfläche des zweiten Polsters (2003b) erstrecken und die relativ zu dem zweiten Polster (2003b) verschiebbar sind; und
eine Einheit (2022), die konfiguriert ist, um mit einer oder beiden der ersten Vielzahl von Kabelschleifen (2019a, 2019a2) und der zweiten Vielzahl von Kabelschleifen (2019b1, 2019b2) wirkgekoppelt zu werden und Kompressions-Expansions-Zyklen auf eines oder beide des ersten und des zweiten Polsters (2003a, 2003b) anzuwenden, indem die effektive Länge der damit wirkgekoppelten Kabelschleifen angepasst wird;
**dadurch gekennzeichnet, dass:**
der Büstenhalter (2001) mindestens eine integrale Tasche (2075) mit einer Öffnung aufweist, die konfiguriert ist, um die Einheit (2022) entfernbar aufzunehmen, sodass die Tasche (2075) mindestens einen Teil der Einheit (2022) während der Verwendung umschließt und sichert;
die Einheit (2022) ein Gehäuse (2023) beinhaltet, wobei die Tasche (2075) bemessen ist, um mindestens einen Teil des Gehäuses (2023) zu umschließen und zu sichern;
das Gehäuse (2023) mindestens eine Batterie (2029), einen Elektromotor (2027), der durch elektrischen Strom gespeist wird, der von der mindestens einen Batterie (2029) geliefert wird, und mindestens eine Leiterplatte mit einer Steuerung und unterstützender Elektronik (2025) umschließt;
der elektrische Motor (2029) konfiguriert ist, um mindestens einen Massageaktor anzutreiben, um die Kompressions-Expansions-Zyklen anzuwenden;
das Gehäuse (2023) der Einheit ferner konfiguriert ist, um einen ersten und einen zweiten Massageaktor (2033a, 2033b), die von dem Elektromotor (2027) angetrieben werden, zu umschließen, wobei der erste Massageaktor (2033a) konfiguriert ist, um lösbar mit der ersten Vielzahl von Kabelschleifen (2019a1, 2019a2) gekoppelt zu werden, um Kompressions-Expansions-Zyklen auf das erste Polster (2003a) anzuwenden, und der zweite Massageaktor (2033b) konfiguriert ist, um lösbar mit der zweiten Vielzahl von Kabelschleifen (2019b1, 2019b2) gekoppelt zu werden, um Kompressions-Expansions-Zyklen auf das zweite Polster (2003b) anzuwenden; und die mindestens eine Tasche eine einzelne Tasche (2075) beinhaltet, die integral mit einer Rückenbahn (2013) des Büstenhalters ist.

2. Gerät zum Abpumpen von Muttermilch gemäß Anspruch 1, wobei:
die Tasche (2075) aus Stoff gebildet ist, der gestrickt oder genäht oder anderweitig an den Büstenhalter angefügt oder in diesen integriert ist.

3. Gerät zum Abpumpen von Muttermilch gemäß Anspruch 1, wobei:
das Gehäuse (2023) ferner eine Vakuumpumpe (2035) umschließt, die durch elektrischen Strom gespeist wird, der von der mindestens einen Batterie (2029) geliefert wird, wobei die Vakuumpumpe (2035) zum Bereitstellen von Saugwirkung an eine externe Saugkopf- und Flaschenanordnung für eine oder beide der linken und der rechten Brust der Benutzerin während der Kompressions-Expansions-Zyklen dient; und/oder
das Gehäuse (2023) ferner ein Magnetventil (2039) umschließt, das konfiguriert ist, um selektiv in die Atmosphäre zu entlüften, um Saugwirkung aufzuheben.

4. Gerät zum Abpumpen von Muttermilch gemäß Anspruch 1, wobei:
das Gehäuse (2023) eine entfernbare Abdeckung (2028) zum Installieren oder Ersetzen der mindestens einen Batterie (2029) umfasst; und/oder die unterstützende Elektronik (2025) eine elektrische Schaltung (2043) umfasst, die konfiguriert ist, um die mindestens eine Batterie (2029) unter Verwendung von Gleichstrom, der von einer externen Gleichstromquelle geliefert wird, zu laden; und/oder
das Gehäuse (2023) ferner eine drahtlose Antenne (2041) und Elektronik umschließt, die konfiguriert sind, um eine drahtlose Kommunikation zwischen der Einheit (2022) und einer anderen Vorrichtung zum Steuern des Betriebs der Einheit (2022) bereitzustellen.

5. Gerät zum Abpumpen von Muttermilch gemäß Anspruch 1, wobei:
der erste und der zweite Massageaktor jeweils einen Drehzugmechanismus (2033a, 2033b) umfassen.

6. Gerät zum Abpumpen von Muttermilch gemäß Anspruch 1, wobei:
der erste und der zweite Massageaktor Drehzugmechanismen beinhalten, die von dem Elektromotor (2027) angetrieben werden (2033a, 2033b); und
die Drehzugmechanismen (2033a, 2033b) mehrteilige Wickelspulen mit Zahnrädern (2093a, 2093b) einsetzen, die mit einem Schneckenrad (2091) in Eingriff stehen, das von dem Elektromotor (2027) drehend angetrieben wird.

7. Gerät zum Abpumpen von Muttermilch gemäß Anspruch 6, wobei mindestens ein Teil der ersten Vielzahl von Kabelschleifen (2019a1, 2019a2) und mindestens ein Teil der zweiten Vielzahl von Kabelschleifen (2019b1, 2019b2) mit Elementen (1113a, 1113b) enden, die von entsprechenden Aufnahmemerkmalen (1115a, 1115b) der mehrteiligen Wickelspulen erfasst werden.

8. Gerät zum Abpumpen von Muttermilch gemäß Anspruch 7, wobei bestimmte Aufnahmemerkmale (1115a, 1115b) der mehrteiligen Wickelspulen in vordefinierten Drehwinkeln für sequenzielle Wickelvorgänge durch die mehrteiligen Wickelspulen voneinander versetzt sind.

9. Gerät zum Abpumpen von Muttermilch gemäß Anspruch 1, das ferner Folgendes beinhaltet:
erstes Schlauchmaterial (2079a1, 2079a2) für die erste Vielzahl von Kabelschleifen (2019a1, 2019a2), wobei sich das erste Schlauchmaterial (2079a1, 2079a2) durch Stoff (2077a), der entlang einer linken Flügelbahn (2021a) des Büstenhalters angeordnet ist, erstreckt oder von diesem gestützt wird; und
zweites Schlauchmaterial (2079b1, 7079b2) für die zweite Vielzahl von Kabelschleifen (2019b1, 2019b2), wobei sich das zweite Schlauchmaterial (2079b1, 7079b2) durch Stoff (2077b), der entlang einer rechten Flügelbahn (2021b) des Büstenhalters angeordnet ist, erstreckt oder von diesem gestützt wird.

10. Gerät zum Abpumpen von Muttermilch gemäß Anspruch 9, wobei:
das Gehäuse (2023) ferner einen ersten Kabelverbinder (2034a), der benachbart zu dem ersten Massageaktor (2033a) angeordnet ist, und einen zweiten Kabelverbinder (2034b), der benachbart zu dem zweiten Massageaktor (2033b) angeordnet ist, umfasst;
das erste Schlauchmaterial (2079a1, 2079a2) an einem Verbinder (2081a) endet, der konfiguriert ist, um selektiv mit dem ersten Kabelverbinder (2034a) zusammenzupassen und zu verriegeln; und
das zweite Schlauchmaterial (2079b1, 7079b2) an einem Verbinder (2081b) endet, der konfiguriert ist, um selektiv mit dem zweiten Kabelverbinder zusammenzupassen und zu verriegeln.

11. Gerät zum Abpumpen von Muttermilch gemäß Anspruch 1, wobei:
das erste Polster (2003a) einen ersten Satz ineinandergreifender perlenartiger Strukturen (3000) beinhaltet, die Durchgänge (2017a1, 2017a2) für Teilstücke der ersten Vielzahl von Kabelschleifen (2019a1, 2019a2) definieren; und
das zweite Polster (2003b) einen zweiten Satz ineinandergreifender perlenartiger Strukturen (3000) beinhaltet, die Durchgänge (2017b1, 2017b2) für Teilstücke der zweiten Vielzahl von Kabelschleifen (2019b1, 2019b2) definieren.

12. Gerät zum Abpumpen von Muttermilch gemäß Anspruch 11, wobei:
die ineinandergreifenden perlenartigen Strukturen (3000) des ersten Satzes innere Federelemente umfassen, die die Expansion der ersten Vielzahl von Kabelschleifen (2019a1, 2019a2) unterstützen; und
die ineinandergreifenden perlenartigen Strukturen (3000) des zweiten Satzes innere Federelemente umfassen, die die Expansion der zweiten Vielzahl von Kabelschleifen (2019b1, 2019b2) unterstützen.

13. Gerät zum Abpumpen von Muttermilch gemäß Anspruch 11, wobei:
die ineinandergreifenden perlenartigen Strukturen (3000) des ersten Satzes jeweils einen im Allgemeinen röhrenförmigen Körper mit einem flachen bogenförmigen Profil entlang der Länge des röhrenförmigen Körpers beinhalten, wobei der röhrenförmige Körper ein Endteilstück mit größerem Durchmesser umfasst, das sich zu einem Halsabschnitt mit kleinerem Durchmesser, der an einem scheibenartigen Kopfabschnitt endet, erstreckt, und der scheibenartige Kopfabschnitt vorzugsweise in das Endteilstück mit größerem Durchmesser der benachbarten perlenartigen Struktur eingesetzt und mit diesem gesichert ist, um die kontinuierlichen Kanäle (2017a1, 2017a2) aus ineinandergreifenden perlenartigen Strukturen des ersten Satzes zu konstruieren; und
die ineinandergreifenden perlenartigen Strukturen (3000) des zweiten Satzes jeweils einen im Allgemeinen röhrenförmigen Körper mit einem flachen bogenförmigen Profil entlang der Länge des röhrenförmigen Körpers beinhalten, wobei der röhrenförmige Körper ein Endteilstück mit größerem Durchmesser umfasst, das sich zu einem Halsabschnitt mit kleinerem Durchmesser, der an einem scheibenartigen Kopfabschnitt endet, erstreckt, und der scheibenartige Kopfabschnitt vorzugsweise in das Endteilstück mit größerem Durchmesser der benachbarten perlenartigen Struktur eingesetzt und mit diesem gesichert ist, um die kontinuierlichen Kanäle (2017b1, 2017b2) aus ineinandergreifenden perlenartigen Strukturen des zweiten Satzes zu konstruieren.

## Revendications

1. Un appareil d'expression de lait maternel comprenant :
un soutien-gorge (2001) comprenant un premier coussinet (2003a) configuré pour entourer le sein gauche d'un utilisateur et un deuxième coussinet (2003b) configuré pour entourer le sein droit de l'utilisateur ;
une première pluralité de boucles de câble (2019a1, 2019a2) avec des portions qui s'étendent circonférentiellement autour d'une surface extérieure du premier coussinet (2003a) et qui sont déplaçables par rapport au premier coussinet (2003a) ;
une deuxième pluralité de boucles de câble (2019b1, 2019b2) avec des portions qui s'étendent circonférentiellement autour d'une surface extérieure du deuxième coussinet (2003b) et qui sont déplaçables par rapport au deuxième coussinet (2003b) ; et
une unité (2022) configurée pour se coupler de manière opérationnelle à l'une ou l'autre ou à l'une et l'autre de la première pluralité de boucles de câble (2019a, 2019a2) et de la deuxième pluralité de boucles de câble (2019b1, 2019b2) et appliquer des cycles de compression-expansion à l'un ou l'autre ou à l'un et l'autre des premier et deuxième coussinets (2003a, 2003b) en ajustant une longueur effective de boucles de câble couplées de manière opérationnelle à ceux-ci ;
**caractérisé en ce que :**
le soutien-gorge (2001) a au moins une poche intégrante (2075) avec une ouverture qui est configurée pour recevoir de manière à ce qu'elle puisse être enlevée l'unité (2022) de sorte que la poche (2075) renferme et fixe au moins une partie de l'unité (2022) pendant l'utilisation ;
l'unité (2022) comprend un boîtier (2023), la poche (2075) étant dimensionnée pour renfermer et fixer au moins une partie du boîtier (2023) ;
le boîtier (2023) renferme au moins une batterie (2029), un moteur électrique (2027) alimenté par une puissance électrique que lui procure l'au moins une batterie (2029), et au moins une carte de circuit imprimé avec un dispositif de commande et une électronique de support (2025) ;
le moteur électrique (2029) est configuré pour entraîner au moins un actionneur de massage dans le but d'appliquer les cycles de compression-expansion ;
le boîtier (2023) de l'unité est en outre configuré pour renfermer des premier et deuxième actionneurs de massage (2033a, 2033b) entraînés par le moteur électrique (2027), le premier actionneur de massage (2033a) étant configuré pour se coupler de manière libérable à la première pluralité de boucles de câble (2019a1, 2019a2) dans le but d'appliquer des cycles de compression-expansion au premier coussinet (2003a), et le deuxième actionneur de massage (2033b) étant configuré pour se coupler de manière libérable à la deuxième pluralité de boucles de câble (2019b1, 2019b2) dans le but d'appliquer des cycles de compression-expansion au deuxième coussinet (2003b) ; et
l'au moins une poche comprend une poche (2075) unique faisant partie intégrante d'un panneau arrière (2013) du soutien-gorge.

2. Un appareil d'expression de lait maternel selon la revendication 1, dans lequel :
la poche (2075) est formée à partir d'un tissu qui est tricoté ou cousu ou autrement joint au soutien-gorge ou intégré dans celui-ci.

3. Un appareil d'expression de lait maternel selon la revendication 1, dans lequel :
le boîtier (2023) renferme en outre une pompe à vide (2035) alimentée par une puissance électrique que lui procure l'au moins une batterie (2029), la pompe à vide (2035) étant destinée à fournir une aspiration à un ensemble tête d'aspiration et bouteille externe pour l'un ou l'autre ou l'un et l'autre du sein gauche et droit de l'utilisateur pendant les cycles de compression-expansion ; et/ou
le boîtier (2023) renferme en outre une électrovanne (2039) configurée pour une mise à l'air libre sélective dans le but de relâcher l'aspiration.

4. Un appareil d'expression de lait maternel selon la revendication 1, dans lequel :
le boîtier (2023) inclut une porte (2028) pouvant être enlevée pour l'installation ou le remplacement de l'au moins une batterie (2029) ; et/ou
l'électronique de support (2025) inclut une circuiterie électrique (2043) configurée pour charger l'au moins une batterie (2029) en utilisant une puissance CC que lui procure une source de puissance CC externe ; et/ou
le boîtier (2023) renferme en outre une antenne sans fil (2041) et une électronique configurées pour fournir une communication sans fil entre l'unité (2022) et un autre dispositif destiné à commander le fonctionnement de l'unité (2022).

5. Un appareil d'expression de lait maternel selon la revendication 1, dans lequel :
les premier et deuxième actionneurs de massage incluent chacun un mécanisme de traction rotatif (2033a, 2033b).

6. Un appareil d'expression de lait maternel selon la revendication 1, dans lequel :
les premier et deuxième actionneurs de massage comprennent des mécanismes de traction rotatifs entraînés (2033a, 2033b) par le moteur électrique (2027) ; et
les mécanismes de traction rotatifs (2033a, 2033b) emploient des bobines d'enroulement en plusieurs parties avec des engrenages (2093a, 2093b) qui s'interfacent avec un engrenage à vis sans fin (2091) qui est entraîné de façon à pouvoir tourner par le moteur électrique (2027).

7. Un appareil d'expression de lait maternel selon la revendication 6, dans lequel au moins une partie de la première pluralité de boucles de câble (2019a1, 2019a2) et au moins une partie de la deuxième pluralité de boucles de câble (2019b1, 2019b2) se terminent par des éléments (1113a, 1113b) qui sont capturés par des caractéristiques en creux (1115a, 1115b) correspondantes des bobines d'enroulement en plusieurs parties.

8. Un appareil d'expression de lait maternel selon la revendication 7, dans lequel certaines caractéristiques en creux (1115a, 1115b) des bobines d'enroulement en plusieurs parties sont décalées les unes par rapport aux autres à des angles de rotation prédéfinis en vue d'opérations d'enroulement séquentielles par les bobines d'enroulement en plusieurs parties.

9. Un appareil d'expression de lait maternel selon la revendication 1, comprenant en outre :
une première tubulure (2079a1, 2079a2) destinée à la première pluralité de boucles de câble (2019a1, 2019a2), la première tubulure (2079a1, 2079a2) s'étendant à travers ou étant supportée par un tissu (2077a) disposé le long d'un panneau de gauche (2021a) du soutien-gorge ; et
une deuxième tubulure (2079b1, 7079b2) destinée à la deuxième pluralité de boucles de câble (2019b1, 2019b2), la deuxième tubulure (2079b1, 7079b2) s'étendant à travers ou étant supportée par un tissu (2077b) disposé le long d'un panneau de droite (2021b) du soutien-gorge.

10. Un appareil d'expression de lait maternel selon la revendication 9, dans lequel :
le boîtier (2023) inclut en outre un premier connecteur de câble (2034a) disposé de manière adjacente au premier actionneur de massage (2033a) et un deuxième connecteur de câble (2034b) disposé de manière adjacente au deuxième actionneur de massage (2033b) ;
la première tubulure (2079a1, 2079a2) se termine au niveau d'un connecteur (2081a) qui est configuré pour, de manière sélective, s'accoupler au premier connecteur de câble (2034a) et se verrouiller sur celui-ci ; et
la deuxième tubulure (2079b1, 7079b2) se termine au niveau d'un connecteur (2081b) qui est configuré pour, de manière sélective, s'accoupler au deuxième connecteur de câble et se verrouiller sur celui-ci.

11. Un appareil d'expression de lait maternel selon la revendication 1, dans lequel :
le premier coussinet (2003a) comprend un premier jeu de structures en forme de perle d'interverrouillage (3000) qui définissent des passages (2017a1, 2017a2) destinés à des sections de la première pluralité de boucles de câble (2019a1, 2019a2) ; et
le deuxième coussinet (2003b) comprend un deuxième jeu de structures en forme de perle d'interverrouillage (3000) qui définissent des passages (2017b1, 2017b2) destinés à des sections de la deuxième pluralité de boucles de câble (2019b1, 2019b2).

12. Un appareil d'expression de lait maternel selon la revendication 11, dans lequel :
les structures en forme de perle d'interverrouillage (3000) du premier jeu incluent des éléments de ressort intérieurs qui aident à l'expansion de la première pluralité de boucles de câble (2019a1, 2019a2) ; et
les structures en forme de perle d'interverrouillage (3000) du deuxième jeu incluent des éléments de ressort intérieurs qui aident à l'expansion de la deuxième pluralité de boucles de câble (2019b1, 2019b2).

13. Un appareil d'expression de lait maternel selon la revendication 11, dans lequel :
les structures en forme de perle d'interverrouillage (3000) du premier jeu comprennent chacune un corps généralement tubulaire avec un profil en forme d'arc peu profond sur la longueur du corps tubulaire, le corps tubulaire incluant une section de queue de plus grand diamètre qui s'étend jusqu'à une portion de col de plus petit diamètre qui se termine au niveau d'une portion de tête en forme de disque, et la portion de tête en forme de disque étant de préférence insérée dans et fixée avec la section de queue de plus grand diamètre de la structure en forme de perle adjacente pour construire les canaux continus (2017a1, 2017a2) à partir de structures en forme de perle d'interverrouillage du premier jeu ; et
les structures en forme de perle d'interverrouillage (3000) du deuxième jeu comprennent chacune un corps généralement tubulaire avec un profil en forme d'arc peu profond sur la longueur du corps tubulaire, le corps tubulaire incluant une section de queue de plus grand diamètre qui s'étend jusqu'à une portion de col de plus petit diamètre qui se termine au niveau d'une portion de tête en forme de disque, et la portion de tête en forme de disque étant de préférence insérée dans et fixée avec la section de queue de plus grand diamètre de la structure en forme de perle adjacente pour construire les canaux continus (2017b1, 2017b2) à partir de structures en forme de perle d'interverrouillage du deuxième jeu.
